# EUROPEAN PATENT APPLICATION

(11) **EP 4 502 596 A1**
(43) Date of publication of application: **05.02.2025**
(21) Application number: 23780950.4
(22) Date of filing: 30.03.2023
(51) Int. Cl.: G01N 33/15, A61K 31/675, A61K 31/706, A61K 38/12, A61K 45/06, A61P 37/06, G01N 33/50, G01N 33/53

(54) **METHOD FOR EVALUATING QUALITY OF INDUCEMENT INHIBITORY-T-CELL FORMULATION**

(30) Priority: 31.03.2022 JP 2022060165
(71) Applicant: Juntendo Educational Foundation, Tokyo 113-8421 (JP); JUNTEN BIO Co., Ltd., Tokyo 100-0004 (JP)
(72) Inventor: UCHIDA, Koichiro, Tokyo 113-8421 (JP); TAKEDA, Kazuyoshi, Tokyo 113-8421 (JP); MAEHARA, Yui, Tokyo 100-0004 (JP); KAWAI, Fumitaka, Tokyo 100-0004 (JP)
(74) Representative: Engelhard, Markus
(86) International application number: PCT/JP2023/013323
(87) International publication number: WO 2023/190942

(57) **Abstract**

According to the present disclosure, optimization of treatment for a rejection reaction by immune tolerance induction is provided.

According to the present disclosure, there is provided a method for evaluating quality of a medicament for achieving immune tolerance, which is administered to a patient who has undergone living-donor liver transplantation, the method including: (a) a step of mixing and culturing peripheral blood lymphocytes collected from a patient before the living-donor liver transplantation and peripheral blood lymphocytes collected from a donor of the living-donor liver transplantation; (b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient; and (c) a step of measuring cytokine production in steps (a) and (b), in which in a case where the cytokine production in step (b) has changed compared to step (a), the medicament is considered to be a medicament for immune tolerance therapy.

## Description

### Technical Field

Liver transplantation has been widely spread as the ultimate therapy for patients with end-stage liver failure. The first clinical liver transplantation in the world was performed in 1963, and there have been about 300,000 or more cases so far. At present, 25,000 or more cases are counted overseas per year, and about 400 cases are counted in Japan. Development of this liver transplantation is due to advances in surgical procedures, organ preservation, preoperative and postoperative management, and among them, improvement of immunosuppressants used for suppressing rejection reactions after transplantation has largely contributed. The 1-year and 5-year patient survival rates have been dramatically improved to about 35% and 20%, about 70% and 60%, and about 80% and 70%, respectively, by azathioprine (1960s to 70s), cyclosporine (1980s), and tacrolimus (since 1990s) used so far.

### Summary of Invention

### Solution to Problem

In the present disclosure, for induction of so-called immune tolerance in which a graft functions normally even when an immunosuppressant is discontinued, a standard of induced regulatory T cells is defined, a method for evaluating the quality of the induced regulatory T cells is found, and a method for achieving immune tolerance using the induced regulatory T cells is established.

Thus, the technology of the present disclosure avoids treatment with immunosuppressants, which are constantly at risk for side effects of drugs/medicaments such as infectious diseases, carcinogenesis, exacerbation of metabolic diseases, and the like, and provides induction of immune tolerance in which the graft functions normally even when the immunosuppressant is discontinued.

As a result of diligent efforts, the present inventors provide a technology that is optimal for practical use in clinical practice, including an appropriate production method for human patients, regarding cells that induce donor antigen-selective immune tolerance via anti-CD80 and anti-CD86 antibody treatment, as well as suppression molecular mechanisms of the cells, for attenuating the immune rejection reaction in organ transplant patients.

An induced regulatory T cell preparation provided by the present disclosure includes induced regulatory T cells induced by co-culturing patient peripheral blood mononuclear cells with radiation-irradiated donor peripheral blood mononuclear cells in the presence of anti-CD80 antibodies and anti-CD86 antibodies. Components of the induced regulatory T cells included in the preparation of the present disclosure are mononuclear cells mainly including T lymphocytes, and among them, CD4⁺ T cells and CD8⁺ T cells are mainly included. The active ingredients of the preparation of the present disclosure are CD4⁺CD25⁺Foxp3⁺ T cells (regulatory CD4⁺ T cells) and CD8⁺CD45RA⁻ T cells (suppressive CD8⁺ T cells).

When the preparation of the present disclosure is administered to an organ transplant patient, the activation of the effector CD4⁺ T cells and the effector CD8⁺ T cells that have reacted with HLA class I and class II antigens expressed in the donor organ is inhibited, and the effector CD4⁺ T cells and the effector CD8⁺ T cells are induced into new regulatory CD4⁺ T cells and suppressive CD8⁺ T cells. As a result, the immune rejection reaction is continuously attenuated in a donor HLA antigen-specific manner, and the amount of immunosuppressants used can be reduced or withdrawn.

Accordingly, the present disclosure provides the following.

### (Item 1)

A method for evaluating quality of a medicament for achieving immune tolerance, which is administered to a patient who has undergone living-donor liver transplantation, the method including:
(a) a step of mixing and culturing peripheral blood lymphocytes collected from a patient before the living-donor liver transplantation and peripheral blood lymphocytes collected from a donor of the living-donor liver transplantation;
(b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient; and
(c) a step of measuring cytokine production in steps (a) and (b), in which
in a case where the cytokine production in step (b) has changed compared to step (a), the medicament is considered to be a medicament for immune tolerance therapy.

### (Item 2)

The method according to the above-described item, in which the patient has undergone administration of an immunosuppressant.

### (Item 3)

The method according to any one of the above-described items, in which the medicament is an inhibitory factor that inhibits an interaction of CD80 and/or CD86 expressed on a cell surface of a certain cell with CD28 expressed on a cell surface of another cell.

### (Item 4)

The method according to any one of the above-described items, in which the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

### (Item 5)

The method according to any one of the above-described items, in which the immune tolerance therapy for the patient is modified in a case where the cytokine production in step (b) changes compared to step (a).

### (Item 6)

The method according to any one of the above-described items, in which
(i) in a case where pro-inflammatory cytokine production in step (b) increases compared to step (a), an amount of immunosuppressant administered to the patient is increased and/or the immune tolerance therapy for the patient is discontinued; and/or
(ii) in a case where anti-inflammatory cytokine production in step (b) decreases compared to step (a), an amount of immunosuppressant administered to the patient is increased and/or the immune tolerance therapy for the patient is discontinued.

### (Item 7)

The method of any one of the above-described items, in which the peripheral blood lymphocytes are stained with CFSE.

### (Item 8)

The method according to any one of the above-described items, in which in steps (a) and (b), B cells collected from the donor are used instead of peripheral blood lymphocytes collected from the donor.

### (Item 9)

The method according to any one of the above-described items, in which the method is carried out within a period from at least about day 3 to day 6 after the living-donor liver transplantation.

### (Item 10)

The method according to any one of the above-described items, in which the cytokine includes IFNy, TNF, IL-2, IL-12, IL-15, IL-17, IL-18, IL-10, and/or TGFβ.

### (Item A1)

A method for achieving immune tolerance in a patient, the method including:
a step of collecting lymphocytes from a donor by apheresis;
a step of collecting lymphocytes from the patient by apheresis;
a step of subjecting a liver or a part of the liver derived from the donor to living-donor liver transplantation for the patient;
a step of performing, on the patient after the living-donor liver transplantation,
   continuous administration of corticosteroids, antimetabolites, and a first immunosuppressant, and
   immune monitoring for rejection reaction to the living-donor liver transplantation and/or regular liver biopsies;
a step of administering a second immunosuppressant to the patient after the living-donor liver transplantation;
a step of administering a medicament for achieving immune tolerance in the patient to the patient after the administration of the second immunosuppressant; and
a step of gradually reducing dosages of the corticosteroids, the antimetabolites, and the first immunosuppressant.

### (Item A2)

The method according to any one of the above-described items, in which
based on the immune monitoring for the rejection reaction and/or the regular liver biopsies, it is determined whether achievement of immune tolerance is confirmed or whether transition to conventional immunosuppressive therapy is performed,
an overall condition of the patient and/or blood biochemical test values are observed over time for a predetermined period after discontinuation of the administration of the first immunosuppressant, and the discontinuation of the administration of the first immunosuppressant is finally determined by performing a liver biopsy at a time point when a predetermined period has elapsed after the discontinuation of the administration of the first immunosuppressant, and
further, in a case where stable liver function values and a state where no pathologically treatable rejection reaction is confirmed in the liver biopsy continue for a predetermined period or more after the discontinuation of the administration of the first immunosuppressant, it is determined that the patient has achieved operational tolerance.

### (Item A3)

A method for achieving immune tolerance in a patient, the method including:
a step of collecting lymphocytes from a donor by apheresis 3 to 14 days before transplantation;
a step of collecting lymphocytes from the patient by apheresis 1 day before transplantation;
a step of subjecting a liver or a part of the liver derived from the donor to living-donor liver transplantation for the patient;
a step of performing, on the patient after the living-donor liver transplantation,
   continuous administration of corticosteroids, antimetabolites, and calcineurin inhibitors, and
   immune monitoring for rejection reaction to the living-donor liver transplantation and/or regular liver biopsies;
a step of administering 20 to 50 mg/kg of cyclophosphamide to the patient on day 4 to day 6 after the liver transplantation;
a step of administering a medicament for achieving immune tolerance in the patient to the patient at any time point between day 9 and day 11 after the liver transplantation; and
a step of reducing a dosage of the calcineurin inhibitor at least 13 weeks after the transplantation, in which
based on the immune monitoring for the rejection reaction and/or the regular liver biopsies, it is determined whether achievement of immune tolerance is confirmed or whether transition to conventional immunosuppressive therapy is performed,
an overall condition of the patient and/or blood biochemical test values are observed over time for at least 52 weeks after discontinuation of the administration of the calcineurin inhibitor, and the discontinuation of the administration of the calcineurin inhibitor is finally determined by performing a liver biopsy at the time point of at least week 52 after the discontinuation of the administration of the calcineurin inhibitor, and
further, in a case where stable liver function values and a state where no pathologically treatable rejection reaction is confirmed by the liver biopsy continue for more than 52 weeks after the discontinuation of the administration of the calcineurin inhibitor, it is determined that the patient has achieved operational tolerance.

### (Item A4)

The method according to any one of the above-described items, in which the administration of the corticosteroids and antimetabolites to the patient is discontinued at least within 4 weeks after the living-donor liver transplantation.

### (Item A5)

The method according to any one of the above-described items, in which the administration of the corticosteroids and antimetabolites to the patient is discontinued at least within 26 weeks after the living-donor liver transplantation.

### (Item A6)

The method according to any one of the above-described items, in which the administration of the calcineurin inhibitors to the patient is discontinued at least within 78 weeks after the living-donor liver transplantation.

### (Item A7)

The method according to any one of the above-described items, in which the medicament is an inhibitory factor that inhibits an interaction of CD80 and/or CD86 expressed on a cell surface of a certain cell with CD28 expressed on a cell surface of another cell.

### (Item A8)

The method according to any one of the above-described items, in which the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

### (Item B 1)

A method for reducing dosage of an immunosuppressant in immune tolerance therapy, the method including:
(1) a step of administering corticosteroids according to the following schedule of administration at the time of reperfusion, and
   administration after liver transplantation and dosage reduction, termination of the administration within a predetermined period, and termination of the administration using an alternative dosage reduction method in a case where the administration is not terminated within the predetermined period;
(2) a step of administering antimetabolites according to the following schedule of administration after liver transplantation,
   termination of the administration within a predetermined period after liver transplantation,
   termination of the administration using an alternative dosage reduction method in a case where the administration is not terminated within a predetermined period after liver transplantation, and
   if necessary, allowing an increase in dosage with the goal of one week after liver transplantation, and in this case, gradual dosage reduction; and
(3) a step of administering a first immunosuppressant according to the following schedule of
   (3-1) a predetermined period after liver transplantation (dosage adjustment period), in which
   administration is initiated on the day of liver transplantation or day 1 after liver transplantation, targeting each of the following blood trough levels such as
   a predetermined blood trough level for a first predetermined period from the initiation of the administration to after liver transplantation, and

   a predetermined blood trough level for a second predetermined period following the first predetermined period after liver transplantation,
      (3-2) week 26 or later after liver transplantation (dosage reduction period of the first immunosuppressant), in which
      in a first predetermined period after liver transplantation (dosage reduction 1), the administration and dosage are adjusted to maintain a predetermined blood trough level, and
      after the second predetermined period after liver transplantation (dosage reduction 2), the dosage of (dosage reduction 1) is maintained, and a frequency of administration is gradually reduced according to a dosage reduction schedule, and
      (3-3) if necessary, the dosage reduction period of the first immunosuppressant is extended, in which
   each dosage reduction is carried out in a case where it is confirmed that no rejection reaction is present.

### (Item B2)

A method for reducing dosage of an immunosuppressant in immune tolerance therapy, the method including:
(1) a step of administering corticosteroids according to the following schedule of administration of 1,000 mg at the time of reperfusion,
   administration of 20 mg/day from day 1 for one week after liver transplantation,
   dosage reduction by 5 mg/day every week and termination of the administration within 4 weeks after liver transplantation, and
   termination of the administration by week 26 after liver transplantation using an alternative dosage reduction method in a case where the administration is not terminated within 4 weeks after liver transplantation;
(2) a step of administering antimetabolites according to the following schedule of
   administration of 500 mg/day in total divided into two doses per day from day 1 after liver transplantation,
   termination of the administration within 4 weeks after liver transplantation,
   termination of the administration by week 26 after liver transplantation using an alternative dosage reduction method in a case where the administration is not terminated within 4 weeks after liver transplantation, and
   if necessary, allowing an increase in dosage to 1,000 to 2,000 mg/day with the goal of one week after liver transplantation, and in this case, gradual dosage reduction by 500 mg/day; and
(3) a step of administering calcineurin inhibitors according to the following schedule of
   (3-1) a period from day 0 after liver transplantation to week 26 after liver transplantation (calcineurin inhibitor dosage adjustment period), in which
   administration is initiated orally or by continuous intravenous infusion on the day of liver transplantation or day 1 after liver transplantation, targeting each of the following blood trough levels such as
   8 to 12 ng/mL of tacrolimus or 200 to 300 ng/mL of cyclosporine during a period from initiation of administration to week 13 after liver transplantation, and
   5 to 8 ng/mL of tacrolimus or 125 to 200 ng/mL of cyclosporine during a period from week 13 after liver transplantation to week 26 after liver transplantation,
   (3-2) week 26 or later after liver transplantation (calcineurin inhibitor dosage reduction period), in which
   in week 26 after liver transplantation (dosage reduction 1), the administration and dosage are adjusted to maintain a blood trough level of 3 to 5 ng/mL of tacrolimus or 75 to 125 ng/mL of cyclosporine with a frequency of administration to once a day, and
   from week 39 after liver transplantation (dosage reduction 2), the dosage from week 26 after liver transplantation (dosage reduction 1) is maintained, and a frequency of administration is gradually reduced according to a calcineurin inhibitor dosage reduction schedule (dosage reduction 2: three times a week, dosage reduction 3: twice a week, dosage reduction 4: once a week, dosage reduction 5: withdrawal), and
   (3-3) if necessary, the calcineurin inhibitor dosage reduction period is extended for a total of 13 weeks, in which
each dosage reduction is carried out in a case where no rejection reaction is confirmed.

### (Item B3)

The method according to any one of the above-described items, in which the presence of the rejection reaction is determined by
(1) physical signs such as fever or general fatigue, and/or
(2) an increase by two or more times from the most recent visit data in any of blood biochemical tests such as T-Bil, AST, ALT, or γ-GTP.

### (Item C1)

A method for confirming presence or absence of a rejection reaction by regular liver biopsies in immune tolerance therapy, the method including:
a step of performing histological diagnosis on samples obtained by collecting liver tissue for control from a transplanted liver on the day of liver transplantation and samples obtained from regular liver biopsies after liver transplantation; and
a step of determining that "there is a pathologically treatable rejection reaction", immediately initiating treatment for the rejection reaction, and discontinuing immune tolerance therapy, in a case where results of overall evaluation of acute rejection reaction satisfy any of the predetermined discontinuation criteria as a result of the histological diagnosis.

### (Item C2)

A method for confirming presence or absence of a rejection reaction by regular liver biopsies in immune tolerance therapy, the method including:
a step of performing histological diagnosis according to scoring based on Banff criteria (2016) and Venturi (2012) with respect to samples obtained by collecting liver tissue for control from a transplanted liver on the day of liver transplantation and samples obtained from regular liver biopsies at week 26 after liver transplantation and 4 weeks and 52 weeks after dosage reduction 5; and
a step of determining that "there is a pathologically treatable rejection reaction", immediately initiating treatment for the rejection reaction, and discontinuing immune tolerance therapy, in a case where results of overall evaluation of acute rejection reaction satisfy "moderate or higher", results of overall evaluation of chronic rejection reaction satisfy "present", and a score of liver fibrosis satisfy "2 or higher" as a result of the histological diagnosis.

### (Item D1)

A method for achieving immune tolerance in a patient who has undergone living-donor liver transplantation using a medicament for achieving immune tolerance evaluated in quality, the method including:
(a) a step of mixing and culturing peripheral blood lymphocytes collected from the patient before the living-donor liver transplantation and peripheral blood lymphocytes collected from a donor of the living-donor liver transplantation;
(b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient;
(c) a step of measuring cytokine production in steps (a) and (b);
(d) a step of performing, on the patient after the living-donor liver transplantation,
   continuous administration of corticosteroids, antimetabolites, and a first immunosuppressant, and
   immune monitoring for rejection reaction to the living-donor liver transplantation and/or regular liver biopsies;
(e) a step of administering a single dose of a second immunosuppressant to the patient after the living donor liver transplant; and
(f) a step of administering a medicament that has changed cytokine production in step (b) compared to step (a), to the patient after the administration of the second immunosuppressant; and
a step of gradually reducing dosages of the corticosteroids, the antimetabolites, and the first immunosuppressant.

### (Item D2)

The method according to any one of the above-described items, in which the patient has undergone administration of an immunosuppressant.

### (Item D3)

The method according to any one of the above-described items, in which the medicament is an inhibitory factor that inhibits an interaction of CD80 and/or CD86 expressed on a cell surface of a certain cell with CD28 expressed on a cell surface of another cell.

### (Item D4)

The method according to any one of the above-described items, in which the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

### (Item D5)

The method according to any one of the above-described items, in which the immune tolerance therapy for the patient is modified in a case where the cytokine production in step (b) changes compared to step (a).

### (Item D6)

The method according to any one of the above-described items, in which
(i) in a case where pro-inflammatory cytokine production in step (b) increases compared to step (a), an amount of immunosuppressant administered to the patient is increased and/or the immune tolerance therapy for the patient is discontinued; and/or
(ii) in a case where anti-inflammatory cytokine production in step (b) decreases compared to step (a), an amount of immunosuppressant administered to the patient is increased and/or the immune tolerance therapy for the patient is discontinued.

### (Item D7)

The method of any one of the above-described items, in which the peripheral blood lymphocytes are stained with CFSE.

### (Item D8)

The method according to any one of the above-described items, in which in steps (a) and (b), B cells collected from the donor are used instead of peripheral blood lymphocytes collected from the donor.

### (Item D9)

The method according to any one of the above-described items, in which the method is carried out within a period from at least about day 3 to day 6 after the living-donor liver transplantation.

### (Item D10)

The method according to any one of the above-described items, in which the cytokine includes IFNy, TNF, IL-2, IL-12, IL-15, IL-17, IL-18, IL-10, and/or TGFβ.

### (Item E1)

A medicament for achieving immune tolerance administered to a patient who has undergone living-donor liver transplantation, in which quality of the medicament was evaluated by the method according to any one of the above-described items.

### (Item F 1)

A method for reducing a dosage of an immunosuppressant in immune tolerance therapy in a patient who has undergone living-donor liver transplantation using a medicament for achieving immune tolerance evaluated in quality, the method including the steps of the method according to any one of the above-described items.

In the present disclosure, it is intended that the one or more features may be provided in further combination in addition to the specified combination. Still further embodiments and advantages of the present disclosure will be appreciated by those skilled in the art upon reading and understanding the following detailed description as appropriate.

### Brief Description of Drawings

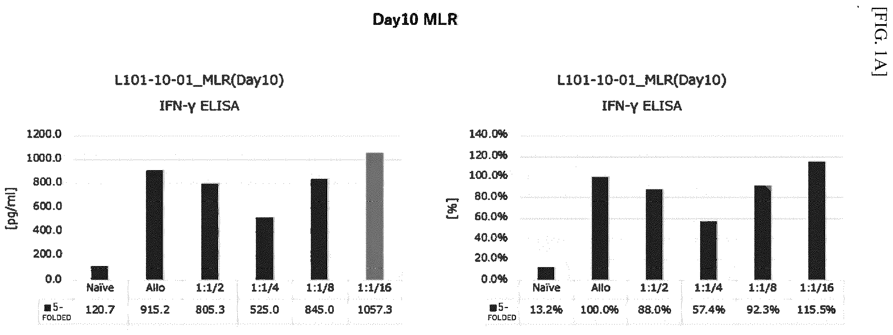

FIG. 1A shows evaluation of immunosuppressive ability of a cell product by MLR. The same number of patient-derived cells and radiation-irradiated donor-derived cells, and the cell products were mixed at a ratio of patient-derived cell number : cell product = 1 : 1/2, 1 : 1/4, 1 : 1/8, or 1 : 1/16 and cultured, and then the IFNg concentration in the culture supernatant was measured by ELISA. FIGS. 1A to 1C respectively show three independent results.

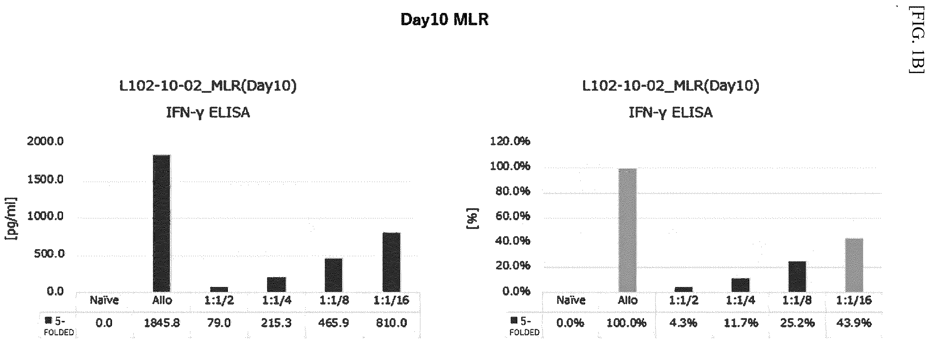

FIG. 1B shows evaluation of immunosuppressive ability of a cell product by MLR. The same number of patient-derived cells and radiation-irradiated donor-derived cells, and the cell products were mixed at a ratio of patient-derived cell number : cell product = 1 : 1/2, 1 : 1/4, 1 : 1/8, or 1 : 1/16 and cultured, and then the IFNg concentration in the culture supernatant was measured by ELISA. FIGS. 1A to 1C respectively show three independent results.

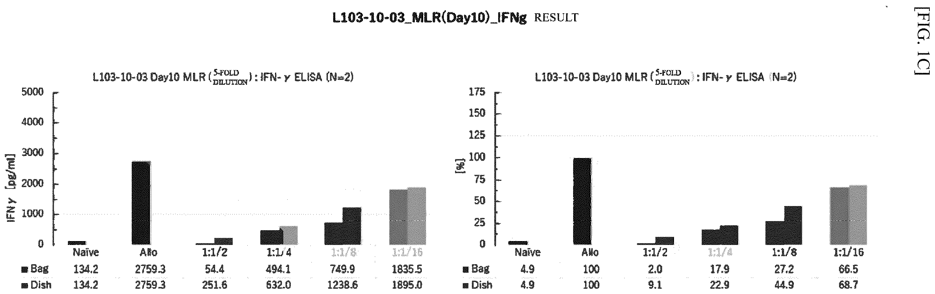

FIG. 1C shows evaluation of immunosuppressive ability of a cell product by MLR. The same number of patient-derived cells and radiation-irradiated donor-derived cells, and the cell products were mixed at a ratio of patient-derived cell number : cell product = 1 : 1/2, 1 : 1/4, 1 : 1/8, or 1 : 1/16 and cultured, and then the IFNg concentration in the culture supernatant was measured by ELISA. FIGS. 1A to 1C respectively show three independent results.

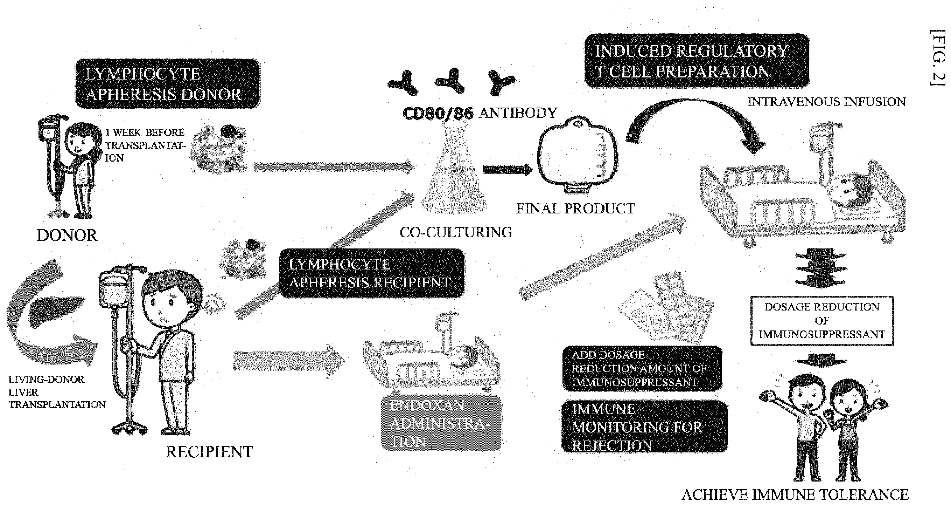

FIG. 2 is a schematic view showing an overall image for achieving dosage reduction of an immunosuppressant for a patient after living-donor liver transplantation, using an induced regulatory T cell preparation according to one embodiment of the present disclosure.

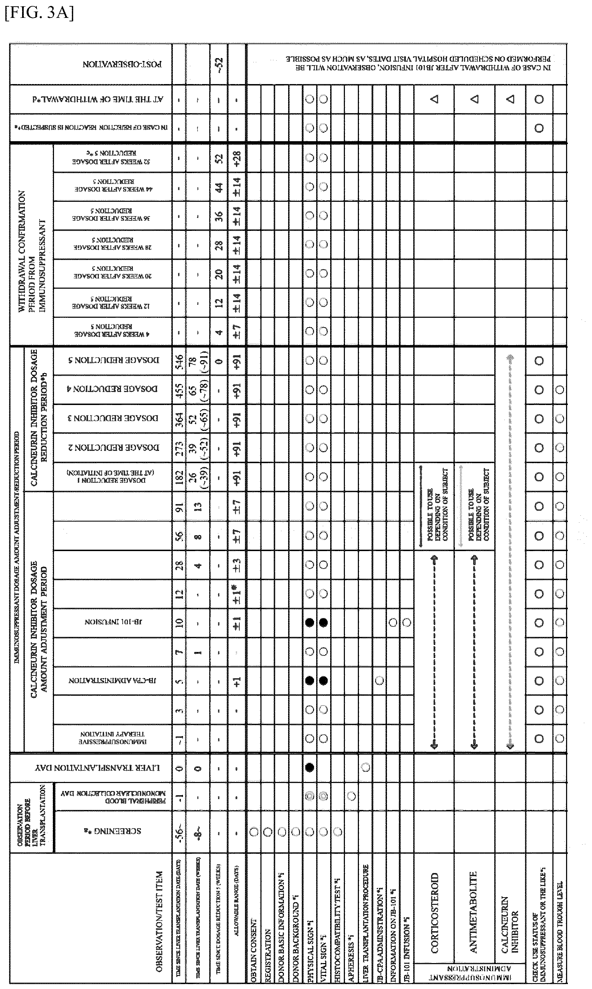

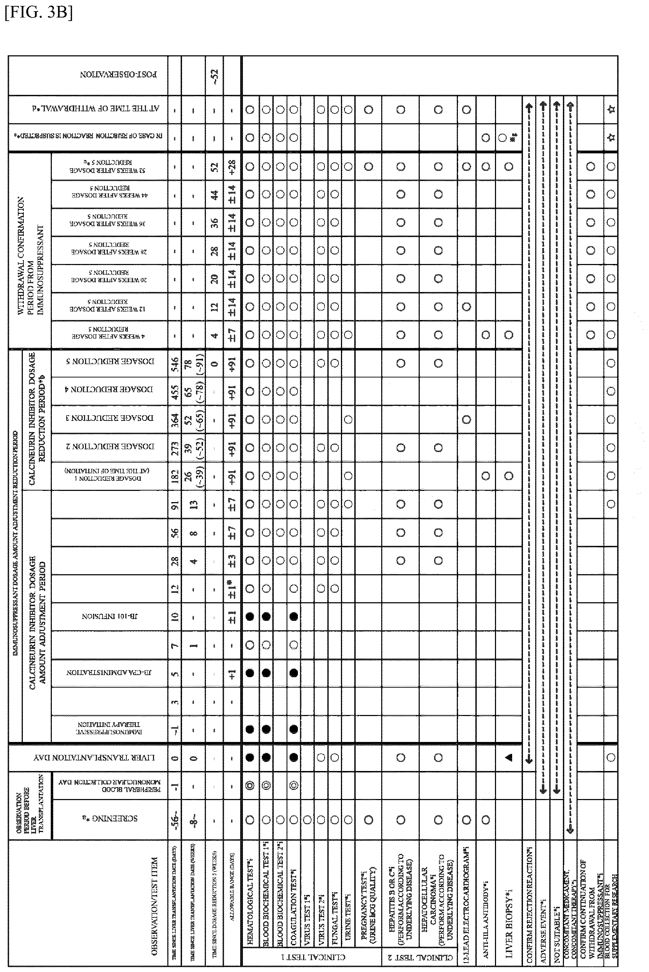

FIG. 3A is an example of a schedule of a patient from living-donor liver transplantation to dosage reduction of an immunosuppressant in a case where dosage reduction of the immunosuppressant of the patient after living-donor liver transplantation is performed using the induced regulatory T cell preparation according to one embodiment of the present disclosure. In the drawing, "JB-101" refers to the induced regulatory T cell preparation of the present disclosure, and "JB-CPA" refers to the injectable Endoxan, respectively. The legend in the drawing is as follows, and is common to FIGS. 3A and 3B. *: The infusion of the induced regulatory T cell preparation of the present disclosure is not allowed to be carried out on the same day. ⊚ : Perform before and after collection of peripheral blood mononuclear cells. However, measurement of the body weight after collection is unnecessary. As the test item before collection of peripheral blood mononuclear cells, test data of the previous day can be used. •: Perform prior to liver transplantation, administration of injectable Endoxan, or infusion of the induced regulatory T cell preparation of the present disclosure. When the immunosuppressive therapy is initiated on the same day as the day of liver transplantation, perform in principle after liver transplantation and before the initiation of administration of immunosuppressants other than steroids. △: Determine dosage or the like by the judgment of a doctor or the like. ▲: Perform at the time of donor liver graft collection (can be done at the back table stage). ☆: If possible, perform based on the judgment of the physician. *a: As the test item at the time of screening, in a case where there is test data within 8 weeks before liver transplantation, the test data can be made available for use under patient's consent. However, in a case where the histocompatibility test, the virus test 1, the virus test 2, the fungal test, the hepatitis B or C test, and the anti-HLA antibody are performed as qualification tests for living-donor liver transplantation, the test data can be made available for use more than 8 weeks before liver transplantation. *b: The timing of dosage reduction can be extended by a total of 13 weeks, but the next dosage reduction is performed at a time interval of 13 weeks or more from the latest dosage reduction time point. *c: In addition to observation of the overall condition of the patient, a blood biochemical test and a liver biopsy are performed, and final confirmation of withdrawal from immunosuppressant is performed. *d: The test items in a case where the administration is discontinued after the administration of injectable Endoxan are indicated. *e: In the case of overlapping with the specified observation date, the test result of the specified observation date is used, and the overlapping items can be omitted. When the test is performed over multiple days, the test is performed within one week in principle. *f: Blood is collected immediately before administration of a specified calcineurin inhibitor, excluding the day of initiation of immunosuppressive therapy, and the blood trough level is measured (blood is collected at least for 12 hours after the previous administration. *g: Liver biopsy and histological diagnosis thereof in a case where a rejection reaction is suspected can be performed in each medical institution where the procedure is carried out.
FIG. 3B is an example of a schedule of a patient from living-donor liver transplantation to dosage reduction of an immunosuppressant in a case where dosage reduction of the immunosuppressant of the patient after living-donor liver transplantation is performed using the induced regulatory T cell preparation according to one embodiment of the present disclosure.

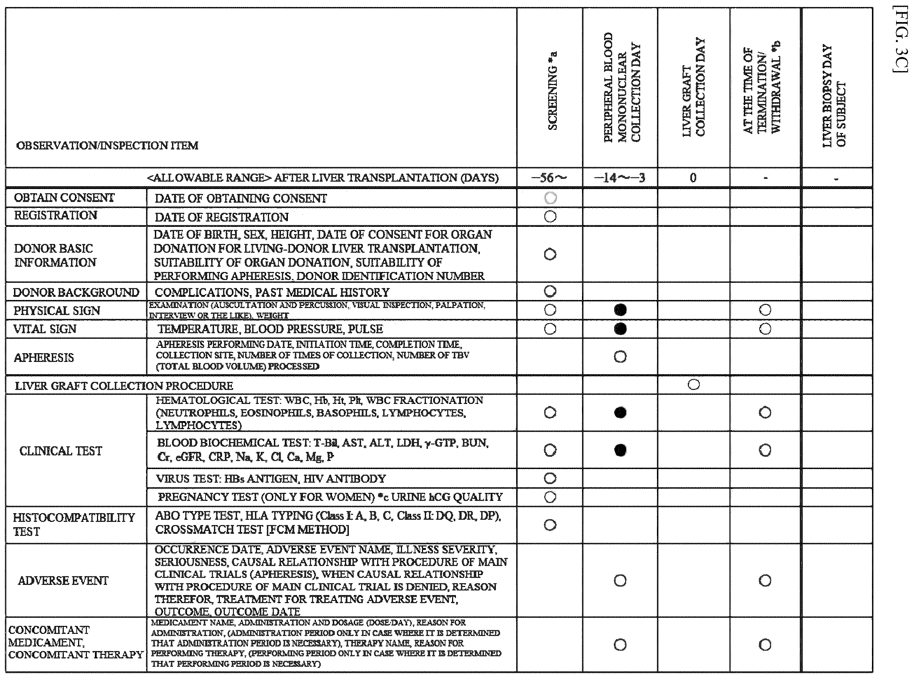

FIG. 3C is an example of a schedule of a donor from living-donor liver transplantation to dosage reduction of an immunosuppressant in a case where dosage reduction of the immunosuppressant of the patient after living-donor liver transplantation is performed using the induced regulatory T cell preparation according to one embodiment of the present disclosure. The legend in the drawing is as follows. •: Perform before initiation and after completion of apheresis. However, measurement of the body weight after completion is unnecessary. *a: As the test item at the time of screening, in a case where there is test data within 8 weeks before transplantation, the test data can be made available for use under donor's consent. However, in a case where the histocompatibility test and the virus test are performed as qualification tests for living-donor liver transplantation, the test data can be made available for use more than 8 weeks before liver transplantation. *b: In a case where the clinical trial is terminated or discontinued after the apheresis procedure (including interruptions), perform the specified test on day 7 after apheresis (allowable range: +7). *c: No test is necessary in a case where 12 months have elapsed after menopause and the uterus or both ovaries are removed.

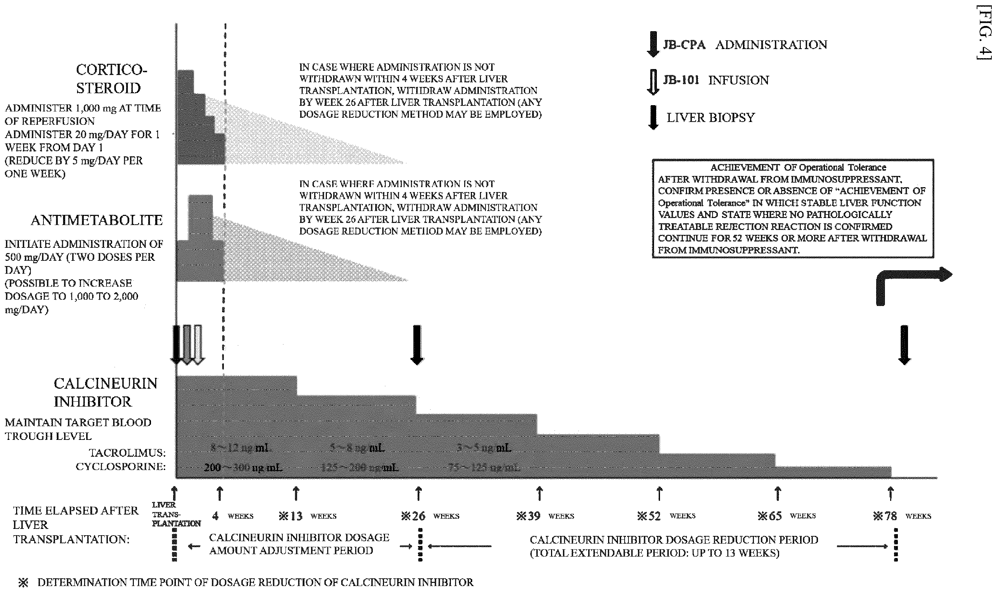

FIG. 4 is a schematic diagram showing an administration/dosage reduction schedule of an immunosuppressant (administration amount adjustment/dosage reduction period of an immunosuppressant) in a case where the induced regulatory T cell preparation according to one embodiment of the present disclosure according to one embodiment is used.

### Description of Embodiments

Hereinafter, the present disclosure will be described while showing the best mode. It is to be understood that throughout the present specification, the singular forms of expression also include the concept of the plural forms thereof, unless otherwise stated. Thus, it is to be understood that the singular article (for example, in English, "a", "an", "the", and the like are used) also includes the concept of the plural forms, unless otherwise stated. It is also to be understood that the terms used in the present specification are used in the sense commonly used in the field unless otherwise noted. Thus, unless defined otherwise, all technical and scientific terms used in the present specification have the same meaning as commonly understood by those skilled in the art to which the present disclosure belongs. In case of conflict, the present specification (including definitions) will take precedence.

The following table provides full names of abbreviations or names or their contents as used in the present specification.

**[Table 1]**

| Abbreviation | English | Japanese (terminology explanation) |
|---|---|---|
| ALT | Alanine aminotransferase | Alanine aminotransferase |
| AST | Aspartate aminotransferase | Aspartate aminotransferase |
| CD4 | Cluster of differentiation 4 | Antigen on cell surface of helper T cell |
| CD8 | Cluster of differentiation 8 | Antigen on cell surface of killer T cell |
| CD25 | Cluster of differentiation 25 | - |
| CD45RA | Cluster of differentiation 45 RA | - |
| CD80 | Cluster of differentiation 80 | Costimulatory factor present on antigen-presenting cells |
| CD86 | Cluster of differentiation 36 | Costimulatory factor present on antigen-presenting cells |
| CYP3A4 | Cvtchmme P450 3A4 | Cytochrome P450 3A4 |
| DNA | Deoxyribonucleic acid | Deoxyribonucleic acid |
| FoxP3 | Forkhead boxprotein P3 | Master transfer factor of regulatory T cell |
| G-CSF | Granulocyte colony stimulating factor | Granulocyte colony stimulating factor |
| GCP | Good Clinical Practice | Ministerial ordinance on standards for performing clinical trials |
| HLA | Human leukocyte antigen | Human leukocyte antigen |
| JB-101 | - | Cell preparation according to the present disclosure including donor antigen-specific induced regulatory T cells cultured ex vivo from autologous peripheral blood |
| JB-CPA | - | Cyclophosphamide hydrate for injection (Endoxan 500mg for Injection) |
| MLR | Mixed lymphocyte reaction | Mixed lymphocyte reaction |
| PCR | Polymerase chain reaction | Polymerase chain reaction |
| RAI | Rejection activity index | - |
| RNA | Ribonucleic acid | Ribonucleic acid |
| T-Bil | Total bilirubin | Total bilirubin |
| γ-GTP | *γ* -Glutamyl transpeptitase | γ-glutamyl transpeptidase |

### (Definition of terms)

In the present specification, it is understood that "about" used in the present specification means ±10% of the subsequent number. In the present specification, even in a case where "about" is not used, it is understood that the subsequent number value may similarly refer to ±10%. In the present specification, the expression "%" means weight/volume (w/v)% unless otherwise specified.

As used in the present specification, "immune tolerance" refers to a state where a specific immune response to a specific antigen is not exhibited or the specific immune response is suppressed. Immune tolerance may mean both or one of a state where an immune cell (particularly a T cell) does not show a specific immune response against a specific antigen or a specific immune response is suppressed, and a state where a human does not show a specific immune response against a specific antigen or a specific immune response is suppressed. This has attracted attention because induction of immune tolerance makes it possible to treat immune rejection reaction and allergies. In the present specification, "anergy" means a state where a cell is unable to respond to the next stimulation under costimulatory conditions due to a lack of costimulatory input when an antigen is presented by an antigen-presenting cell. Thus, as used in the present specification, an "induced regulatory cell" or "anergy cell" refers to a cell in which immune tolerance has occurred (immune unresponsive), and an "induced regulatory T cell" or "anergy T cell" is a T cell in which immune tolerance has occurred (immune unresponsive) and is not activated, and also includes a T cell that is unresponsive when the cell encounters the same antigen again. In the present specification, "immune tolerance induced PBMCs (or T cells)", "induced regulatory T cells", and "anergic PBMCs (or T cells)" have the same meaning.

In the present specification, "subject" (in English, may be expressed as "object") or "patient" includes domestic animals (for example, bovine, ovine, feline, canine, and equine), primates (for example, humans and non-human primates such as monkeys), rabbits, and rodents (for example, mice and rats). In certain embodiments, the subject or patient is a human.

The target disease of the preparation of the present disclosure is immune rejection reaction to a donor organ of patients who have undergone allogenic organ transplantation between living bodies. As an example, in addition to living body liver transplantation, immune rejection reaction associated with living body kidney transplantation, living body lung transplantation, and the like is targeted.

According to the preparation of the present disclosure, in a subject (recipient) to which cells having anergy induced to a specific antigen have been administered by an inhibitory factor such as an antibody capable of inhibiting the interaction of CD80 and/or CD86 with CD28, the state of immune tolerance to the specific antigen is maintained for at least a certain period (for example, several months, one year, or three or more years) even after the cells having anergy induced have disappeared from the subject, and permanent immune tolerance (infectious immune tolerance) can be achieved. This means that anergy is newly induced in other cells in which anergy can be induced in the living body of the subject (recipient) to which the cells in which the anergy is induced are administered, that is, infectious immune tolerance is induced.

In the present specification, "medicament", "agent", or "factor" (any of these corresponds to "agent" in English) is used interchangeably in a broad sense and may be any substance or other elements (for example, energy such as light, radioactivity, heat, and electricity) as long as it is possible to achieve the intended purpose. Such substances include, for example, but are not limited to, proteins, polypeptides, oligopeptides, peptides, polynucleotides, oligonucleotides, nucleotides, nucleic acids (including, for example, cDNA, DNA such as genomic DNA, and RNA such as mRNA), polysaccharides, oligosaccharides, lipids, low-molecular-weight organic molecules (for example, hormones, ligands, information transmitters, other organic low-molecular-weight compounds, molecules synthesized by combinatorial chemistry, low-molecular-weight molecules that can be utilized as pharmaceuticals (for example, a low-molecular-weight ligand or the like)), and complex molecules thereof.

In the present specification, "inhibitory factor" refers to all kinds of low-molecular-weight molecules, proteins, nucleic acids, lipids, sugars, and the like that can inhibit a predetermined action (for example, interactions, signal transduction, and the like). Without wishing to be bound by any particular theory, in the present disclosure, anergy is induced in T cells by blocking the interaction of CD80 and/or CD86 on the cell surface with CD28 and inhibiting the CD28 costimulatory signal. In the present disclosure, the inhibitory factor used to block the interaction of CD80 and/or CD86 with CD28 is selected from the group consisting of low-molecular-weight molecules, proteins, nucleic acids, lipids, sugars, and combinations thereof. In one aspect, the protein is an antibody or a variant thereof, or a cell surface molecule or a variant thereof. In another aspect, a variant of the antibody is an antigen-binding fragment. In another aspect, the variant of the cell surface molecule is a fusion protein. In another aspect, the inhibitory factor is selected from the group consisting of anti-CD80 antibodies, anti-CD86 antibodies, bispecific antibodies against CD80 and CD86, anti-CD28 antibodies or antigen-binding fragments thereof, CTLA4-Ig fusion proteins, and CD28-Ig fusion proteins. In another aspect, the CTLA4-Ig fusion protein is abatacept or belatacept. In addition, it is also conceivable to use a factor that indirectly inhibits the interaction (for example, an inhibitory factor of a signal upstream or downstream of signal transduction) in combination.

"Antibody" in a broad sense in the present specification refers to a molecule or a population thereof capable of specifically binding to a particular epitope on an antigen. "Antibody" in a broad sense in the present specification may be a full-length antibody (that is, an antibody having an Fc region) or an antibody lacking an Fc region. The antibody lacking the Fc region only needs to be able to bind to the antigen of interest, and examples of such an antibody include, but are not limited to, a Fab antibody, a F(ab')₂ antibody, a Fab' antibody, a Fv antibody, and a scFv antibody. The antibody may be any type of antibody, that is, an immunoglobulin known in the field. In exemplary embodiments, the antibody is an antibody of the isotype IgA, IgD, IgE, IgG, or IgM class. In exemplary embodiments, the antibodies described in the present specification include one or more alpha, delta, epsilon, gamma, and/or mu heavy chains. In exemplary embodiments, the antibodies described in the present specification include one or more kappa or light chains. In exemplary embodiments, the antibody is an IgG antibody and is one of four human subclasses, that is, IgG1, IgG2, IgG3, and IgG4. Examples of the antibody that is contemplated for use in the present disclosure include an antibody derived from a camelid (for example, VHH antibodies), an antibody derived from a shark (for example, single-chain antibodies), a peptibody, a nanobody (single domain antibody), a minibody, a multispecific antibody (for example, bispecific antibodies, diabodies, triabodies, tetrabodies, and tandem di-scFV, tandem tri-scFv), which are known in the field. For example, refer to Kortt et al., Biomol Eng. 2001, Vol. 18: p. 95 to 108 (2001), and Todorovska et al., J Immunol Methods. Vol. 248: p. 47 to 66 (2001). The antibody also includes modified and unmodified antibodies. In the antibody-modified product, the antibody may be bound to various molecules such as polyethylene glycol. An antibody-modified product can be obtained by subjecting an antibody to chemical modification using a known method. Also refer to Biochemistry (2016), Vol. 88, No. 3, p. 380 to 385 for artificially created antibodies and various methods of modification/alteration of antibodies.

In the present specification, "antibody" in a narrow sense refers to an immunoglobulin or a population thereof capable of specifically binding to a specific epitope on an antigen, and a variant thereof is referred to as "antibody variant". "Antibody" in the narrow sense in the present specification may be a full-length antibody (that is, an antibody having an Fc region) and "antibody variant" in the present specification may be a variant lacking the Fc region of the antibody. Thus, in the present specification, an antibody in the narrow sense may also be referred to as a full-length antibody, and a variant of an antibody may also be referred to as a variant of a full-length antibody. The variant lacking the Fc region only needs to be able to bind to the antigen of interest, and examples of such a variant include, but are not limited to, a Fab antibody, a F(ab')₂ antibody, a Fab' antibody, a Fv antibody, and a scFv antibody. The variant of the antibody also includes modified and unmodified antibodies. In the antibody-modified product, the antibody may be bound to various molecules such as polyethylene glycol. An antibody-modified product can be obtained by subjecting an antibody to chemical modification using a known method.

In the present specification, "polyclonal antibody" can be generated, for example, by administering an immunogen containing an antigen of interest to mammals (for example, rat, mouse, rabbit, cow, and monkey), birds, or the like, in order to induce the production of a polyclonal antibody specific to the antigen. Administration of the immunogen may include the injection of one or more immunizing agents and, if desired, adjuvants. The adjuvant may be used to increase the immune response, and may include Freund's adjuvant (complete or incomplete), a mineral gel (such as aluminum hydroxide), a surfactant (such as lysolecithin), or the like. Immunization protocols are known in the field and may be carried out by any method of eliciting an immune response, depending on the selected host organism (Protein Experiment Handbook, YODOSHA CO., LTD. (2003): 86-91).

In the present specification, "monoclonal antibody" includes a case where individual antibodies constituting a population are substantially identical antibodies corresponding to a single epitope except for antibodies having mutations that may occur naturally in a small amount. Alternatively, the individual antibodies constituting a population may be substantially identical except for antibodies having mutations that may occur naturally in a small amount. Monoclonal antibodies are highly specific and are different from normal polyclonal antibodies, typically including different antibodies corresponding to different epitopes and/or typically including different antibodies corresponding to the same epitope. In addition to the specificity thereof, monoclonal antibodies are useful in that the monoclonal antibodies can be synthesized from hybridoma cultures that are not contaminated by other immunoglobulins. The expression "monoclonal" may refer to the feature of being obtained from a substantially homogeneous antibody population, but does not mean that the antibody must be produced in any particular manner. For example, monoclonal antibodies may be produced by a method similar to the hybridoma method as described in "Kohler G, Milstein C., Nature, 1975 Aug 7; 256 (5517): 495-497". Alternatively, monoclonal antibodies may be produced by methods similar to recombinant methods as described in U.S. Patent No. 4816567. Alternatively, monoclonal antibodies may be isolated from phage antibody libraries using methods similar to those described in "Clackson et al., Nature. 1991 Aug 15;352(6336):624-628" or "Marks et al., J Mol Biol. 1991 Dec 5;222(3):581-597". Alternatively, monoclonal antibodies may be produced by the method described in "Protein Experiment Handbook, YODOSHA CO., LTD. (2003): 92-96".

In one embodiment of the present disclosure, "chimeric antibody" links, for example, a variable region of an antibody and a constant region of an antibody between heterologous organisms, and can be constructed by a genetic recombination technology. The mouse-human chimeric antibody can be produced, for example, by the method described in "Roguska et al., Proc Natl Acad Sci U S A. 1994 Feb 1;91(3):969-973". The basic method for producing mouse-human chimeric antibodies, for example, links the mouse leader sequence and the variable region sequence present in the cloned cDNA to the sequence encoding human antibody constant region which is already present in mammalian cell expression vectors. Alternatively, the mouse leader sequence and the variable region sequence which are present in the cloned cDNA may be linked to the sequence encoding human antibody constant region, and then may be linked to a mammalian cell expression vector. Fragments of the human antibody constant region can be of the H chain constant region of any human antibody and the L chain constant region of a human antibody, for example, Cγ1, Cy2, Cy3, or Cy4 for human H chain and Cλ. or Cκ for L chain, respectively.

In one embodiment of the present disclosure, "humanized antibody" is an antibody that has, for example, one or more CDRs derived from a non-human species, and a framework region (FR) derived from a human immunoglobulin, and further a constant region derived from a human immunoglobulin, and binds to a desired antigen. Humanization of antibodies can be performed using a variety of technologies known in the field (Almagro et al., Front Biosci. 2008 Jan 1;13:1619-1633). For example, CDR grafting (Ozaki et al., Blood, 1999 Jun1; 93 (11): 3922-3930.), Re-surfacing (Roguska et al., Proc Natl Acad Sci US A. 1994 Feb 1;91(3):969-973), FR shuffling (Damschroder et al., Mol Immunol. 2007 Apr;44(11):3049-3060. Epub 2007 Jan 22), or the like can be mentioned. To alter (preferably improve) antigen binding, amino acid residues in the human FR region may be replaced with corresponding residues from the CDR donor antibody. This FR substitution can be carried out by methods well known in the field (Riechmann et al., Nature. 1988 Mar 24; 332 (6162): 323-327). For example, FR residues that are important for antigen binding may be identified by modelling the interaction of CDRs with FR residues. Alternatively, sequence comparison may identify abnormal FR residues at specific positions.

In one embodiment of the present disclosure, "human antibody" is, for example, an antibody in which a region including a variable region and a constant region of a heavy chain, and a variable region and a constant region of a light chain, which constitute the antibody, is derived from a gene encoding human immunoglobulin. Examples of the main production method include a transgenic mouse method for producing a human antibody and a phage display method. In the transgenic mouse method for producing a human antibody, when a functional human Ig gene is introduced into a mouse in which an endogenous Ig is knocked out, a human antibody having various antigen-binding abilities is produced instead of a mouse antibody. Furthermore, by immunizing this mouse, a human monoclonal antibody can be obtained by a conventional hybridoma method. For example, the human monoclonal antibody can be produced by the method described in "Lonberg et al., Int Rev Immunol.1995;13(1):65-93". The phage display method is a system in which a foreign gene is expressed as a fusion protein on the N-terminal side of a coating protein (g3p, g10p, and the like) of a fibrous phage such as M13 or T7, which is typically one of E. coli viruses, not to lose infectivity of the phage. For example, the phage display can be produced by the method described in "Vaughan et al., Nat Biotechnol. 1996 Mar;14(3):309-314".

In one embodiment of the present disclosure, in a case where the preparation of the present disclosure is produced, cells obtained from a subject (patient) to whom the composition or preparation of the present disclosure is administered or cells derived from the subject can be used. In one embodiment of the present disclosure, the preparation of the present disclosure can also be produced using an antigen generated by the subject itself that elicits an immune response, for example, an antigen derived from the subject, which is an antigen generated by the subject itself that causes an autoimmune disease in a subject with autoimmune disease. In one embodiment of the present disclosure, the preparation of the present disclosure can also be produced using antigens that are not derived from a subject and are foreign antigens that can elicit an immune response. In one embodiment of the present disclosure, the preparation of the present disclosure can also be produced using an antigen-containing material that is not derived from a subject, which is any substance or collection of substances that contains an antigen that is not derived from a subject, and examples thereof include cells, cell populations, tissues, and the like that express an antigen that is not derived from a subject.

It is known that in a subject or patient who has undergone a transplant of an organ, tissue, or cell, the subject or patient's immune system causes a transplant immune rejection reaction that attacks, damages, or destroys the transplanted organ, tissue, or cell, and in one embodiment of the present disclosure, the composition or preparation of the present disclosure can also cause immune tolerance in such a case.

### (Preferred embodiments)

Although a description of preferred embodiments is set forth below, it is to be understood that this embodiment is illustrative of the present disclosure and that the scope of the present disclosure is not limited to such preferred embodiments. It should be understood that those skilled in the art can also easily make alterations, modifications, and the like within the scope of the present disclosure with reference to the following preferred examples. For these embodiments, those skilled in the art may combine any embodiments as appropriate in view of the description in the present specification. It is also understood that the following embodiments of the present disclosure may be used alone or in combination.

### <Induced Regulatory T Cell Preparation>

According to one aspect, the present disclosure provides an induced regulatory T cell preparation, use methods such as administration and dosage thereof, and a technology for evaluating the quality of a medicament for achieving immune tolerance administered to a patient who has undergone living-donor liver transplantation using the induced regulatory T cell preparation and use methods thereof.

In one embodiment, the induced regulatory T cells of the present disclosure are induced by co-culturing patient peripheral blood mononuclear cells with the radiation-irradiated donor peripheral blood mononuclear cells in the presence of an anti-CD80 antibodies and an anti-CD86 antibodies. Components of the induced regulatory T cells of the present disclosure are mononuclear cells mainly including T lymphocytes, and among them, CD4⁺T cells and CD8⁺T cells are mainly included. In one embodiment, the active ingredients of the induced regulatory T cells of the present disclosure are CD4⁺CD25⁺FoxP3⁺ T cells (regulatory CD4⁺ T cells) and CD8⁺CD45RA⁻ T cells (suppressive CD8⁺ T cells). Without being bound by theory, when the induced regulatory T cells of the present disclosure are administered to an organ transplant patient, the activation of the effector CD4⁺ T cells and the effector CD8⁺ T cells that have reacted with HLA class I and class II antigens expressed in the donor organ is inhibited, and the effector CD4⁺ T cells and the effector CD8⁺ T cells are induced into new regulatory CD4⁺ T cells and suppressive CD8⁺ T cells. As a result, it is expected that the immune rejection reaction is continuously attenuated in a donor HLA antigen-specific manner, and the amount of immunosuppressants used can be reduced or withdrawn.

The induced regulatory T cells of the present disclosure can be produced in Cell Processing Room, Research Center, Juntendo University Graduate School of Medicine, which is a cell culture processing facility.

In one embodiment, the induced regulatory T cells of the present disclosure can be a liquid preparation containing nucleated cells containing autologous induced regulatory T cells, in one bag (100 mL) of primary packaging, for example, about 5.0 × 10⁵ or more, about 5.0 × 10⁶ or more, about 5.0 × 10⁷ or more, about 5.0 × 10⁸ or more, or about 5.0 × 10⁹ or more. In one embodiment, the induced regulatory T cells of the present disclosure can be induced by co-culturing subject peripheral blood mononuclear cells with the radiation-irradiated donor peripheral blood mononuclear cells in the presence of an anti-CD80 antibodies and an anti-CD86 antibodies.

In one embodiment, the storage method and effective period of the induced regulatory T cells of the present disclosure are not particularly limited, so long as the effect of inducing immune tolerance is achieved. For example, the storage method can be about 8 ± 3°C, about 7 ± 3°C, about 6 ± 3°C, about 5 ± 3°C, or about 4 ± 3°C. The effective period can be, for example, within about 36 hours, within about 30 hours, within about 24 hours, within about 18 hours, within about 12 hours, or within about 6 hours after completion of the primary packaging.

In one embodiment, the primary packaged bag for storage of induced regulatory T cells of the present disclosure can be stored in a secondary packaging container in one bag/pouch and further stored in a final packaging container with a water absorbent sheet in one bag/pouch. A label describing "for treatment", "serial number", "product name", "number of contained cells", "expiration date", "storage condition", and the like can be attached to the primary packaging.

In the present disclosure, an example of the "medicament for achieving immune tolerance" may be an "inhibitory factor that inhibits the interaction of CD80 and/or CD86 expressed on the cell surface of a certain cell with CD28 expressed on the cell surface of another cell", and examples thereof may include, but are not limited to, anti-CD80 antibodies, anti-CD86 antibodies, bispecific antibodies thereof, and inhibitory factors against CD80 and/or CD86 (for example, abatacept or belatacept, or the induced regulatory T cells described in the present specification).

In the present disclosure, in one example, the anti-CD80 antibody and the anti-CD86 antibody described in WO2019/245037 (PCT/JP2019/024752), WO2014/186193 (PCT/US2014/037195) and the like can be used for the "anti-CD80 antibody" and the "anti-CD86 antibody", respectively. For example, as the anti-CD80 antibody and the anti-CD86 antibody, a chimeric anti-CD80 antibody and a chimeric anti-CD86 antibody may be used, respectively. For example, as the anti-CD80 antibody and the anti-CD86 antibody, a human anti-CD80 antibody and a human anti-CD86 antibody may be used, respectively. The anti-human CD80 antibody may be of the subclass IgG1. In addition, the anti-human CD86 antibody may also be of the subclass IgG1. The plasmids encoding the anti-CD80 antibody and the anti-CD86 antibody may be produced in-house, or may be outsourced (for example, TPG Biologics, Inc., Taiwan) to make them expressed in CHO cells, and the antibodies which were produced and purified can be used. Antibodies other than the anti-CD80 and anti-CD86 antibodies specifically described in WO2019/245037 (PCT/JP2019/024752), WO2014/186193 (PCT/US2014/037195) and the like may also be used.

In another embodiment, "anti-CD80 antibody" and "anti-CD86 antibody" are interchangeable with any inhibitory factor capable of inhibiting the interaction of CD80 and/or CD86 with CD28. Examples of the inhibitory factors include, in addition to the anti-CD80 antibodies and the anti-CD86 antibodies, bispecific antibodies against CD80 and CD86, anti-CD28 antibodies or antigen-binding fragments thereof, CTLA4-Ig fusion proteins (for example, abatacept or belatacept), and CD28-Ig fusion proteins. Belatacept is available, for example, from Bristol-Myers Squibb, New York, NY (final concentration 10 µg/ml to 40 µg/ml).

In one embodiment of the present disclosure, Endoxan can be administered as a pretreatment for an induced regulatory T cell infusion therapy to acquire immune tolerance following liver transplantation. As an example of Endoxan, the following types can be used.
(1) Name
   · Product name: Injectable Endoxan 500 mg
   · Generic name: Cyclophosphamide Hydrate for Injection
(2) Composition
   The "Cyclophosphamide Hydrate for Injection (hereinafter, Cyclophosphamide)" administered as a preconditioning medicament contains 534.5 mg of cyclophosphamide hydrate (equivalent to 500 mg as cyclophosphamide anhydrous) per vial.
(3) Storage method
   Store at 28°C (refrigerator)
(4) Packaging and display
   The vials containing 534.5 mg cyclophosphamide hydrate are stored in a white box, with 1 vial/box. A label describing "for treatment", "serial number", "identification code", "expiration date", "storage condition", and the like can be attached to the enclosure (vial) and the outer box (white box).

### <Quality evaluation>

According to one aspect of the present disclosure, there is provided a method for evaluating quality of a medicament for achieving immune tolerance, which is administered to a patient who has undergone living-donor liver transplantation, the method including:
(a) a step of mixing and culturing peripheral blood lymphocytes collected from a patient before the living-donor liver transplantation and peripheral blood lymphocytes collected from a donor of the living-donor liver transplantation;
(b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient; and
(c) a step of measuring cytokine production in steps (a) and (b), in which in a case where the cytokine production in step (b) has changed compared to step (a), the medicament is considered to be a medicament for immune tolerance therapy.

In one embodiment, the patient in the method of the present disclosure may or may not have undergone administration of an immunosuppressant in advance.

In one embodiment, the medicament of the present disclosure can be an inhibitory factor that inhibits the interaction of CD80 and/or CD86 expressed on the cell surface of a certain cell with CD28 expressed on the cell surface of another cell, and examples of such a medicament may include belatacept, abatacept, and induced regulatory T cell preparations to be described below. In one embodiment, the medicament of the present disclosure can be an induced regulatory T cell preparation, and in this case, the induced regulatory T cell preparation can be obtained by co-culturing the peripheral blood lymphocytes derived from the patient and the donor of the living-donor liver transplantation in the presence of CD80 antibodies and/or CD86 antibodies. In one embodiment, the induced regulatory T cells that can be used in the quality evaluation method of the present disclosure include the induced regulatory T cells described in other parts of the present disclosure.

In one embodiment of the present disclosure, in a case where cytokine production in step (b) has changed compared to step (a), the immune tolerance therapy for the patient can be modified, and for example, (i) in a case where pro-inflammatory cytokine production in step (b) increases compared to step (a), the amount of immunosuppressant administered to the patient can be increased and/or the immune tolerance therapy for the patient can be discontinued. In another embodiment, (ii) in a case where the anti-inflammatory cytokine production in step (b) decreases compared to step (a), the amount of the immunosuppressant administered to the patient can be increased, and/or immune tolerance therapy for the patient can be discontinued. In one embodiment, the increase or decrease in cytokine production can include a case where a detectable difference occurs when comparing step (a) and step (b).

In one embodiment of the present disclosure, peripheral blood lymphocytes may be stained with CFSE. In another embodiment, in steps (a) and (b), B cells collected from the donor can also be used instead of peripheral blood lymphocytes collected from the donor.

In one embodiment of the present disclosure, the immunosuppressant is not particularly limited, but for example, a calcineurin inhibitor can be used, and examples of the calcineurin inhibitor include tacrolimus and cyclosporine.

In one embodiment of the present disclosure, the quality evaluation method of the present disclosure is for achieving immune tolerance in a patient who has undergone living-donor liver transplantation, and can be performed at any time point, for example, within at least about 2 days, within about 4 days, within about 6 days, or within about 8 days after living-donor liver transplantation, or before living-donor liver transplantation. In addition, in one embodiment, the method of the present disclosure can also be performed multiple times.

In one embodiment, cytokines may include IFNy, TNF, IL-2, IL-12, IL-15, IL-17, IL-18, IL-10, TGFβ, and the like, among which inflammatory cytokines may include IL-17, IL-18, and the like, and anti-inflammatory cytokines may include IL-10, TGFβ, and the like.

Measurement of cytokine production used in a method for evaluating the quality of a medicament for achieving immune tolerance administered to a patient who has undergone living-donor liver transplantation can be performed, for example, as follows using an ELISA method.

### 1.1. Confirmation of sample

For the sample used for ELISA, information of the culture supernatant is confirmed.

### 1.2. Preparation of ELISA reagent

All reagents and samples used are allowed to return to room temperature prior to use.

### 1.2.1. Preparation of Wash Buffer

The Wash Buffer Concentrate is diluted 25 times with distilled water to prepare a Wash Buffer.

### 1.2.2. Preparation of Calibrator Diluent

Calibrator Diluent RD5P is diluted 5-fold with distilled water to prepare Calibrator Diluent RD5P (dilution ratio 1 : 5).

### 1.2.3. Preparation of Standard Stock Solution (10,000 pg/mL)

Human IFN-y Standard is melted with the amount of distilled water indicated on the label of the vial, then stirred gently and allowed to stand for at least 15 minutes to prepare Standard Stock Solution (10,000 pg/mL).

### 1.2.4. Preparation of standard dilution series

900 µL of Calibrator Diluent RD5P (dilution ratio 1 : 5) is collected and mixed with 100 µL of Standard Stock Solution (10,000 pg/mL) to adjust the Standard Stock Solution (1,000 pg/mL). Standard Stock Solution (1,000 pg/mL) is diluted double using Calibrator Diluent RD5P (dilution ratio 1 : 5) to prepare a 2-fold dilution series of standard solutions. A polypropylene tube is used for preparing the dilution series solution. Standard Stock Solution (1,000 pg/mL) is used as the highest concentration point of the standard, and Calibrator Diluent RD5P (dilution ratio 1 : 5) is used as the 0 standard (Blank).

### 1.2.5. Preparation of diluted sample

The sample is diluted with Calibrator Diluent RD5P (dilution ratio 1 : 5). The homogeneously mixed sample is set in a centrifuge, centrifuged at 460 G for 5 minutes at 4°C, and the supernatant is used for dilution.

### 1.2.6. Preparation of substrate solution

Color Reagent A and Color Reagent B are mixed in equal amounts to prepare a Substrate Solution. The prepared Substrate Solution is stored in the dark and used within 15 minutes after preparation. The required amount of the prepared solution is calculated from 200 µL/well.

### 1.2.7. Assay

1) Primary Reaction
   a. A plate layout is created.
   b. The necessary Microplate Strips is set in the Plate Frame (hereinafter, Assay Plate).
   c. 100 µL/well of Assay Diluent RD1-63 is added to the Assay plate.
   d. According to the plate layout in a., 100 µL/well of Calibrator Diluent RD5P (dilution ratio 1 : 5) is added as a standard dilution series, sample, and blank. This operation is completed within 15 minutes after the addition of Assay Diluent RD1-63.
   e. The Assay plate is covered with a plate seal, placed in a constant temperature shaking culture machine (room temperature) set at 25°C, and incubated stationary for 2 hours.
2) Secondary Reaction
   a. The added solution is removed from each well of the Assay plate, and washed with 400 µL/well of Wash Buffer at least 4 times.
   b. The Assay plate is flipped over the paper wiper to remove any remaining Wash Buffer and add 200 µL/well of IFN-y Conjugate. The Assay plate is covered with a new plate seal, placed in a constant temperature shaking culture machine (room temperature) set at 25°C, and incubated stationary for 2 hours.
3) Color Development
   a. The added solution is removed from each well of the Assay plate, and washed with 400 µL/well of Wash Buffer at least 4 times.
   b. The Assay plate is flipped over the paper wiper to remove any remaining Wash Buffer and add 200 µL/well of Substrate Solution. The Assay plate is covered with a new plate seal, placed in a constant temperature shaking culture machine (room temperature) set at 25°C, and incubated stationary for 30 minutes in the dark.
   c. After adding 50 µL/well of Stop Solution to the Assay plate, the plate is gently tapped to be stirred, and it is confirmed that the color of the solution in the well changes uniformly from blue to yellow.
4) Measurement
   a. The OD of each well is measured within 30 minutes using a microplate reader set at 450 nm/570 nm.
   b. After completion of the measurement, it is confirmed that a calibration curve is created on software and the IFN-y amount of each sample is calculated, and the calibration curve is overwritten and stored.
   c. The measurement results are printed and the data is exported.

### 2. Analysis

Calculation and graph creation are performed using the data of the microplate reader.

### 2.1. Comparison of IFN-y concentration of each sample (pg/mL)

The IFN-y amount (pg/mL) of each sample calculated on the software of the microplate reader is compared.

### 2.2. Comparison of proportion (%) of IFN-y amount using Allo as index

Using the IFN-y amount of the sample calculated on the software of the microplate reader, the proportion (%) of the IFN-y amount of each sample in a case where the IFN-y amount of Allo is set to 100% is calculated and compared.

The calculation formula is as follows. IFN-y amount of each sample (Naive and sample No. (1) to (4))/IFN-y amount of Allo × 100

In a case where the immune reaction between the recipient and the donor is weak, it is expected to be difficult to compare the proportion (%) of the IFN-y amount using Allo as an index. Therefore, co-culture is performed using each cell suspension of which concentration is adjusted to 4.0 × 10⁶ cells/mL and a culture medium, and the results calculated from these culture supernatants are treated as a reference.

In one embodiment of the present disclosure, the quality evaluation method of the present disclosure can be performed, for example, by utilizing a mixed lymphocyte reaction (MLR), and MLR can be performed, as an example, as follows.

### Confirmation of Sample

Information on the MLR sample (the following items are examples) is recorded.
· Sample identification information (ID, serial number, and the like)
· Receiving container
. Cell number
· Liquid amount

### 1.1.1. Donor frozen cell and recipient frozen cell

Information on frozen and stored donor cells and recipient cells is recorded.

### 1.2. The donor frozen cells and the recipient frozen cells are thawed.

1) To 1 to 2 frozen cryotubes to be thawed, 13 mL of the culture medium is dispensed into 15 mL centrifuge tubes.
2) The frozen cryotubes are immersed in a water bath at 37°C and pulled up once semi-thawed.
3) The thawed cells are transferred to 13 mL of culture medium dispensed in 1).
4) A 15 mL centrifuge tube is set in a centrifuge, and centrifuged at 460 G for 5 minutes at 4°C.
5) The supernatant is removed and the pellet is suspended in an appropriate amount of culture medium.
6) A small amount of the cell suspension is collected, diluted 2 to 100 times with a trypan blue solution, and then subjected to cell counting.
7) The viable cell concentration is calculated based on the number counted in 6). The calculation formula is as follows. (number of viable cells in total of 4 compartments)/4 × dilution factor × 10⁴ = viable cell concentration (cells/mL)

### 1.1.1. Adjustment of Cell Concentration

1) The cell product can be adjusted to any cell concentration using a culture medium based on the cell information of the MLR sample (cell product) provided from the CPC. For example, in one embodiment, the concentration can be adjusted to a total cell concentration of 2.0 × 10⁵ cells/mL, 4.0 × 10⁵ cells/mL, 2.0 × 10⁶ cells/mL, 4.0 × 10⁶ cells/mL, 2.0 × 10⁷ cells/mL, 4.0 × 10⁷ cells/mL, or the like.
2) The recipient cell and the donor cell can be adjusted to any viable cell concentration using the culture medium based on the cell count result. For example, in one embodiment, the concentration can be adjusted to a viable cell concentration of 2.0 × 10⁵ cells/mL, 4.0 × 10⁵ cells/mL, 2.0 × 10⁶ cells/mL, 4.0 × 10⁶ cells/mL, 2.0 × 10⁷ cells/mL, 4.0 × 10⁷ cells/mL, or the like.

### 1.2. Initiation of Co-culture

1) In one embodiment, a cell mixture mixed at the ratio in Table 2 is prepared using each cell suspension of which concentration is adjusted to 2.0 × 10⁶ cells/mL and the culture medium (Plate No. 1). The preparation is carried out under the following conditions.
2) In one embodiment, a cell mixture mixed at the ratio in Table 2 is prepared using each cell suspension of which concentration is adjusted to 4.0 × 10⁶ cells/mL and the culture medium (Plate No. 2). The preparation is carried out under the following conditions. A sample name is assigned to each mixing ratio, and hereinafter, the sample is identified by the sample name.

The order of mixing may be any order. For example, in one embodiment, the order can be recipient cells → cell products → culture media → donor cells.

1 well of each cell suspension is independently seeded at 250 µL/well into 3 wells of a 96-well round-bottom plate.

**[Table 2]**

| Sample | | ① | ② | ③ | ④ |
|---|---|---|---|---|---|
| No. | Name | Recipient cell | Cell product | culture medium | Doner cell |
| - | Naïve | 100 µL | - | 150 µL | - |
| - | Allo | 100 µL | - | 50 µL | 100 µ1 |
| (1) | 1:1/2 | 100 µL | 50 µL | - | 100 µL |
| (2) | 1:1/4 | 100 µL | 25 µL | 25 µL | 100 µL |
| (3) | 1:1/8 | 100 µL | 12.5 µL | 37.5 µL | 100 µL |
| (4) | 1:1/16 | 100 µL | 6.25 µL | 43.75 µL | 100 µL |
| - | A+D | - | 100 µL | 50 µL | 100 µL |

3) Culturing is initiated in CO₂ incubator.

### 1.1. Collection of culture supernatant (termination of co-culture)

Collection of culture supernatant is performed after 5 days from the initiation of culturing.
1) The 96-well round-bottom plate under culture is taken out from the CO₂ incubator, and it is confirmed that no abnormality such as contamination occurs, and the culturing is terminated.
2) For each sample, 220 µL of culture supernatant is collected into a 1.5 mL Eppendorf tubes independently for each well.
   * Be careful not to touch the bottom surface with the tip end of the pipette to avoid collecting cell pellets
3) The collected culture supernatant is used as a sample for ELISA. When ELISA is not performed on the day of collection, the sample is frozen and stored in a biomedical freezer.

MLR as described above is a method for evaluating reactivity of lymphocytes to non-self antigens, and thus is useful for evaluating the quality of the induced regulatory T cells of the present disclosure that induce immune tolerance. For example, by performing this test, it is possible to predict how much the immune reaction against the transplanted organ of the patient can be suppressed after administration of the induced regulatory T cells of the present disclosure. In one embodiment, the lymphocytes derived from the patient and the donor used in the MLR test can be used by taking a part of those obtained in the process of producing the induced regulatory T cells of the present disclosure, and thus the test can be carried out without additional burden on the patient and the donor for sample collection.

In one embodiment of the present disclosure, the ELISA method described above can be performed according to the flow of the instruction manual of the conventionally known method or kit, and also for the MLR method, in addition to the matters described in the present specification, a part or all of the features of the flow of the instruction manual of the conventionally known method or kit can be appropriately used.

### <Immune tolerance by induced regulatory T cell preparation>

In one embodiment of the present disclosure, the immune tolerance inducibility (efficacy) after infusion of the induced regulatory T cells of the present disclosure can be evaluated for the patients who have undergone living-donor liver transplantation by using the presence or absence of achievement of operational tolerance as an index.

Therefore, according to one aspect of the present disclosure, there is provided a method for achieving immune tolerance in a patient, the method including: a step of collecting lymphocytes from a donor by apheresis; a step of collecting lymphocytes from the patient by apheresis; a step of subjecting a liver or a part of the liver derived from the donor to living-donor liver transplantation for the patient; a step of performing, on the patient after the living-donor liver transplantation, continuous administration of corticosteroids, antimetabolites, and a first immunosuppressant, and immune monitoring for rejection reaction to the living-donor liver transplantation and/or regular liver biopsies; a step of administering a second immunosuppressant to the patient after the living-donor liver transplantation; a step of administering a medicament for achieving immune tolerance in the patient to the patient after the administration of the second immunosuppressant; and a step of gradually reducing dosages of the corticosteroids, the antimetabolites, and the first immunosuppressant.

In one embodiment of the present disclosure, based on immune monitoring for the rejection reaction and/or regular liver biopsies, it is possible to determine whether achievement of immune tolerance is confirmed or whether transition to conventional immunosuppressive therapy is performed. In one embodiment, an overall condition of the patient and/or blood biochemical test values can be observed over time for a predetermined period after discontinuation of the administration of the first immunosuppressant, and the discontinuation of the administration of the first immunosuppressant can be finally determined by performing a liver biopsy at a time point when a predetermined period has elapsed after the discontinuation of the administration of the first immunosuppressant. In addition, in another embodiment, in a case where stable liver function values and a state where no pathologically treatable rejection reaction is confirmed in the liver biopsy continue for a predetermined period or more after the discontinuation of the administration of the first immunosuppressant, it is also possible to determine that the patient has achieved operational tolerance.

In addition, according to another aspect of the present disclosure, there is provided a method for achieving immune tolerance in a patient, the method including: a step of collecting lymphocytes from a donor by apheresis 3 to 14 days before transplantation; a step of collecting lymphocytes from the patient by apheresis 1 day before transplantation; a step of subjecting a liver or a part of the liver derived from the donor to living-donor liver transplantation for the patient; a step of performing, on the patient after the living-donor liver transplantation, continuous administration of corticosteroids, antimetabolites, and calcineurin inhibitors, and immune monitoring for rejection reaction to the living-donor liver transplantation and/or regular liver biopsies; a step of administering 20 to 50 mg/kg of cyclophosphamide to the patient on day 4 to day 6 after the liver transplantation; a step of administering a medicament for achieving immune tolerance in the patient to the patient at any time point between day 9 and day 11 after the liver transplantation; and a step of reducing a dosage of the calcineurin inhibitor at least 13 weeks after the transplantation, in which based on the immune monitoring for the rejection reaction and/or the regular liver biopsies, it is determined whether achievement of immune tolerance is confirmed or whether transition to conventional immunosuppressive therapy is performed, an overall condition of the patient and/or blood biochemical test values are observed over time for at least 52 weeks after discontinuation of the administration of the calcineurin inhibitor, and the discontinuation of the administration of the calcineurin inhibitor is finally determined by performing a liver biopsy at the time point of at least week 52 after the discontinuation of the administration of the calcineurin inhibitor, and further, in a case where stable liver function values and a state where no pathologically treatable rejection reaction is confirmed by the liver biopsy continue for more than 52 weeks after the discontinuation of the administration of the calcineurin inhibitor, it is determined that the patient has achieved operational tolerance.

In one embodiment of the present disclosure, the corticosteroid is not particularly limited, but for example, methylprednisolone can be used. In one embodiment, the antimetabolite is not particularly limited, but for example, mycophenolate mofetil can be used.

In one embodiment of the present disclosure, the first immunosuppressant is not particularly limited, but for example, a calcineurin inhibitor can be used, and examples of the calcineurin inhibitor include tacrolimus and cyclosporine. In one embodiment, the second immunosuppressant is not particularly limited, but examples thereof include cyclophosphamide.

In one embodiment of the present disclosure, the administration of the corticosteroids and antimetabolites to the patient can be discontinued at least within 4 weeks after living-donor liver transplantation. In another embodiment, the administration of the corticosteroids and antimetabolites to the patient can be discontinued at least within 26 weeks after living-donor liver transplantation.

In one embodiment of the present disclosure, the administration of the calcineurin inhibitor to the patient can be discontinued at least within 78 weeks after living-donor liver transplantation.

In one embodiment of the present disclosure, achievement of operational tolerance can be defined as a state where a patient is withdrawn from an immunosuppressant by week 78 (at most by week 91) after liver transplantation, and thereafter, a state where a stable liver function value and a pathologically treatable rejection reaction by a liver biopsy cannot be confirmed continues for 52 weeks or more after the withdrawal from immunosuppressants.

In one embodiment of the present disclosure, the transition in the dosage of the calcineurin inhibitor from the initiation of dosage reduction to the time of withdrawal when the dosage is reduced 26 weeks or more after liver transplantation according to the method for dosage reduction/withdrawal of an immunosuppressant, and the duration of withdrawal of the corticosteroids and the antimetabolites can be used as secondary evaluation items of efficacy. Furthermore, in other embodiments, the safety of immunosuppressants, apheresis, cyclophosphamide can also be evaluated.

In one embodiment of the present disclosure, the immune tolerance by the induced regulatory T cell preparation of the present disclosure can be targeted for an end-stage liver failure patient undergoing living-donor liver transplantation, and includes a pre-liver transplantation observation period, a dosage adjustment/dosage reduction period of the immunosuppressant, and a withdrawal confirmation period from the immunosuppressant.

In one embodiment of the present disclosure, in the pre-liver transplantation observation period, after consent is obtained, a screening test can be performed, peripheral blood mononuclear cells can be collected from the donor by 14 to 3 days before liver transplantation, and peripheral blood mononuclear cells can be collected from the subject by a lymphoapheresis method on the day before liver transplantation. The collected subject peripheral blood mononuclear cells are cultured for 10 days together with the donor peripheral blood mononuclear cells (antigen) inactivated by radiation-irradiation and the anti-CD80/anti-CD86 monoclonal antibodies, and the induced regulatory T cells of the present disclosure can be induced.

In one embodiment, during the dosage adjustment/dosage reduction period of the immunosuppressant, 40 mg/kg of cyclophosphamide (reduced to 20 mg/kg as necessary) can be administered on day 5 after liver transplantation for the purpose of temporarily reducing the in-vivo lymphocytes. Thereafter, the induced regulatory T cells of the present disclosure are infused into a subject on day 10 after liver transplantation, and immune tolerance inducibility and safety by the induced regulatory T cells of the present disclosure until week 78 (up to week 91) after liver transplantation can be evaluated. In one embodiment, the subject can be managed under hospitalization for at least 4 weeks after liver transplantation.

In one embodiment, the immunosuppressant used in the method of the present disclosure is not particularly limited, but for example, a medicament (corticosteroids, antimetabolites, calcineurin inhibitors, and the like) conforming to that after normal liver transplantation can be used, and dosage reduction can be performed according to the method for reducing or withdrawing the immunosuppressant of the present disclosure.

It is preferable to discontinue the administration of the corticosteroids and the antimetabolite mycophenolate mofetil with the goal of within 4 weeks after the liver transplantation. However, in a case where the administration cannot be discontinued within 4 weeks after the liver transplantation according to the judgment of a doctor or the like depending on the condition of the subject or the like, the administration of the calcineurin inhibitor can be discontinued before the initiation of dosage reduction of the calcineurin inhibitor. Immunosuppression can be maintained with a calcineurin inhibitor alone with tacrolimus as the first choice from week 4 after liver transplantation, and after week 26 after liver transplantation, the dosage can be reduced gradually according to the dosage reduction schedule of the calcineurin inhibitor, and finally, withdrawal from the immunosuppressant by acquisition of immune tolerance can be performed. The administration/dosage reduction schedule of the immunosuppressant according to one embodiment is shown in FIG. 4.

In one embodiment, during the withdrawal confirmation period from the immunosuppressant, the overall condition of the subject, blood biochemical test value, and the like are observed over time for 52 weeks after the withdrawal from the immunosuppressant, and the presence or absence of achievement of operational tolerance can be confirmed at week 52 after the withdrawal from the immunosuppressant by liver biopsy or the like.

### <Method for performing living-donor liver transplantation>

In one embodiment, the liver graft collection procedure of the donor and the liver transplantation procedure of the subject, and the postoperative management procedure of the donor and the subject can conform to the standard liver transplantation procedure and the postoperative management procedure performed in each medical institution where the procedure is carried out. In one embodiment, the following test and evaluation are performed on day 0, which is the day of living-donor liver transplantation.

### Pre-infusion treatment of induced regulatory T cell preparation (Endoxan administration)

In one embodiment, Endoxan can be used as a preconditioning to reduce lymphocytes in a subject prior to infusion of an induced regulatory T cell preparation. After liver transplantation, a doctor or the like examines whether or not it is possible to administer Endoxan depending on the overall condition or the like of the subject, and in a case where it is determined that the subject is eligible, Endoxan can be administered to the subject via intravenous infusion over 23 hours on day 5 after liver transplantation. In one embodiment, the standard dosage of Endoxan can be about 40 mg/kg, but since the agent is a medicament of liver metabolism, the dosage can also be adjusted within the range of about 20 to about 40 mg/kg while confirming the transition of liver function after liver transplantation. Since a case where the myelosuppressive effects of leukopenia reduction and neutropenia reduction significantly appears after administration of Endoxan has been reported, in other embodiments, a doctor or the like can adjust the dosage with reference to past cases.

In one embodiment, the following measures can also be taken as a response to the side effects of Endoxan.

For nausea and vomiting assumed as a side effect of the preconditioning medicament (Endoxan), prophylactically, 9.9 mg (equivalent to 50 mg of methylprednisolone) of dexamethasone can be administered on day 1 (before administration of Endoxan), 125 mg of Aprepitant (or 150 mg of Fosaprepitant) can be administered, and 8 mg (equivalent to 40 mg of methylprednisolone) of a 5-HT₃ receptor antagonist can be administered on day 24. Since Aprepitant (or Fosaprepitant) is a substrate of CYP3A4 and has mild to moderate CYP3A4 inhibition (dosage-dependent) and inducing action, it is preferable to avoid administration since it may affect the blood concentration of tacrolimus. In addition, administration of uromitexan for suppressing the expression of urinary system disorders (Hemorrhagic cystitis, dysuria, and the like) and other prophylactic support therapies (prophylactic administration of antibiotics and the like) can also be performed.

In one embodiment, a G-CSF preparation can also be used in a case where the neutrophil count is less than 1,000/mm³ after Endoxan administration. In a case where the induced regulatory T cell preparation could not be administered after the administration of Endoxan, even when the neutrophil count is not less than 1,000/mm³, the G-CSF preparation can be administered in a case where a decreasing tendency is observed and the neutrophil count is predicted to be less than 1,000/mm³. However, when the neutrophil count increases to 5,000/mm³ or more, it is preferable to reduce the dosage or discontinue the administration depending on the symptoms.

### <Infusion of induced regulatory T cell preparation>

In one embodiment, the doctor or the like examines whether or not the infusion of the induced regulatory T cell preparation can be performed from the overall condition or the like of the subject after the administration of Endoxan, and in a case where it is determined that the subject is eligible, the induced regulatory T cell preparation can be infused to the subject. A blood transfusion filter can be used for the infusion of the induced regulatory T cell preparation, and the constituent cells of the induced regulatory T cell preparation are removed by the leukocyte removal filter, and thus it is preferable not to use the filter.

In one embodiment, infusion can be initiated at a low rate, increase the rate of administration and complete the administration of the induced regulatory T cell preparation in about 30 minutes while confirming the condition of the subject. During the administration of the induced regulatory T cell preparation, the doctor or the like can constantly observe the condition of the subject and immediately interrupt the administration and examine whether or not the administration can be resumed in a case where a symptom such as a decrease in blood pressure, which is difficult to control, appears. In the case of resuming, in a case where a situation requiring interruption occurs again, the subsequent administration can be discontinued.

### <Dosage reduction/withdrawal of immunosuppressant, determination of presence or absence of achievement of Operational tolerance, and reduction/withdrawal method of each immunosuppressant (dosage adjustment/dosage reduction period of immunosuppressant)>

According to one aspect of the present disclosure, there is provided a method for reducing dosage of an immunosuppressant in immune tolerance therapy, the method including:
(1) a step of administering corticosteroids according to the following schedule of administration at the time of reperfusion, and
   administration after liver transplantation and dosage reduction, termination of the administration within a predetermined period, and termination of the administration using an alternative dosage reduction method in a case where the administration is not terminated within the predetermined period;
(2) a step of administering antimetabolites according to the following schedule of administration after liver transplantation,
   termination of the administration within a predetermined period after liver transplantation,
   termination of the administration using an alternative dosage reduction method in a case where the administration is not terminated within a predetermined period after liver transplantation, and
   if necessary, allowing an increase in dosage with the goal of one week after liver transplantation, and in this case, gradual dosage reduction; and (3) a step of administering a first immunosuppressant according to the following schedule of
   (3-1) a predetermined period after liver transplantation (dosage adjustment period), in which
   administration is initiated on the day of liver transplantation or day 1 after liver transplantation, targeting each of the following blood trough levels such as
   a predetermined blood trough level for a first predetermined period from the initiation of the administration to after liver transplantation, and
   a predetermined blood trough level for a second predetermined period following the first predetermined period after liver transplantation,
   (3-2) week 26 or later after liver transplantation (dosage reduction period of the first immunosuppressant), in which
   in a first predetermined period after liver transplantation (dosage reduction 1), the administration and dosage are adjusted to maintain a predetermined blood trough level, and
   after the second predetermined period after liver transplantation (dosage reduction 2), the dosage of (dosage reduction 1) is maintained, and a frequency of administration is gradually reduced according to a dosage reduction schedule, and
   (3-3) if necessary, the dosage reduction period of the first immunosuppressant is extended, in which
each dosage reduction is carried out in a case where it is confirmed that no rejection reaction is present.

According to another aspect of the present disclosure, there is provided a method for reducing dosage of an immunosuppressant in immune tolerance therapy, the method including:
(1) a step of administering corticosteroids according to the following schedule of administration of 1,000 mg at the time of reperfusion,
   administration of 20 mg/day from day 1 for one week after liver transplantation,
   dosage reduction by 5 mg/day every week and termination of the administration within 4 weeks after liver transplantation, and
   termination of the administration by week 26 after liver transplantation using an alternative dosage reduction method in a case where the administration is not terminated within 4 weeks after liver transplantation;
(2) a step of administering antimetabolites according to the following schedule of
   administration of 500 mg/day in total divided into two doses per day from day 1 after liver transplantation,
   termination of the administration within 4 weeks after liver transplantation,
   termination of the administration by week 26 after liver transplantation using an alternative dosage reduction method in a case where the administration is not terminated within 4 weeks after liver transplantation, and
   if necessary, allowing an increase in dosage to 1,000 to 2,000 mg/day with the goal of one week after liver transplantation, and in this case, gradual dosage reduction by 500 mg/day; and
(3) a step of administering calcineurin inhibitors according to the following schedule of
   (3-1) a period from day 0 after liver transplantation to week 26 after liver transplantation (calcineurin inhibitor dosage adjustment period), in which
   administration is initiated orally or by continuous intravenous infusion on the day of liver transplantation or day 1 after liver transplantation, targeting each of the following blood trough levels such as
   8 to 12 ng/mL of tacrolimus or 200 to 300 ng/mL of cyclosporine during a period from initiation of administration to week 13 after liver transplantation, and
   5 to 8 ng/mL of tacrolimus or 125 to 200 ng/mL of cyclosporine during a period from week 13 after liver transplantation to week 26 after liver transplantation,
   (3-2) week 26 or later after liver transplantation (calcineurin inhibitor dosage reduction period), in which
   in week 26 after liver transplantation (dosage reduction 1), the administration and dosage are adjusted to maintain a blood trough level of 3 to 5 ng/mL of tacrolimus or 75 to 125 ng/mL of cyclosporine with a frequency of administration to once a day, and
   from week 39 after liver transplantation (dosage reduction 2), the dosage from week 26 after liver transplantation (dosage reduction 1) is maintained, and a frequency of administration is gradually reduced according to a calcineurin inhibitor dosage reduction schedule (dosage reduction 2: three times a week, dosage reduction 3: twice a week, dosage reduction 4: once a week, dosage reduction 5: withdrawal), and
   (3-3) if necessary, the calcineurin inhibitor dosage reduction period is extended for a total of 13 weeks, in which
each dosage reduction is carried out in a case where no rejection reaction is confirmed.

FIG. 4 shows an administration and dosage reduction schedule of the immunosuppressant in the dosage adjustment and dosage reduction period of the disease suppressor according to one embodiment of the present disclosure.

In one embodiment, after liver transplantation, the following immunosuppressive therapy can be performed. The doctor or the like can determine the dosage reduction/withdrawal of each immunosuppressant after confirming that no rejection reaction is observed at the timing of considering the dosage reduction/withdrawal. In addition, when a side effect caused by an immunosuppressant is developed, the amount can be reduced as necessary.

### Corticosteroid (methylprednisolone and the like)

In one embodiment, in principle, preferably, the administration is performed according to the judgment of a doctor or the like depending on the condition of the subject from the day of liver transplantation, and the administration is discontinued by week 4 after liver transplantation. When the administration cannot be terminated within 4 weeks after the liver transplantation, the dosage can be gradually reduced and the administration can be terminated by week 26 after the liver transplantation. Hereinafter, a reference administration/dosage reduction schedule is shown.

1,000 mg of methylprednisolone is administered at the time of reperfusion, and 20 mg/day of methylprednisolone is administered for one week from the next day of liver transplantation (day 1). The dosage of the corticosteroid is reduced by 5 mg/day per one week, and the administration is discontinued with the goal of within 4 weeks after the liver transplantation.

### Antimetabolite (mycophenolate mofetil)

In one embodiment, in principle, preferably, the administration is performed according to the judgment of a doctor or the like depending on the condition of the subject from the next day of liver transplantation, and the administration is discontinued by week 4 after liver transplantation. When the administration cannot be terminated within 4 weeks after the liver transplantation, the dosage can be gradually reduced and the administration can be terminated by week 26 after the liver transplantation. Hereinafter, a reference administration/dosage reduction schedule is shown.

From the next day of liver transplantation (day 1), 500 mg/day of mycophenolate mofetil (divided into 2 times a day) is administered. The dosage is increased to 1,000 to 2,000 mg/day with the goal of day 7 for about one week, and then reduced gradually every 500 mg/day, and administration is discontinued with the goal of within 4 weeks after liver transplantation.

### Calcineurin inhibitor (tacrolimus cyclosporine)

In one embodiment, tacrolimus is used as the calcineurin inhibitor as the first choice, and when tacrolimus cannot be used due to side effects or the like, cyclosporine is preferably used as the second choice. However, when a doctor or the like determines that it is necessary to temporarily stop the calcineurin inhibitor due to infection or the like, the calcineurin inhibitor can be discontinued for an appropriate period of time.

The dosage reduction method of the calcineurin inhibitor can be set with reference to the administration and dosage in the case of bone marrow transplantation of the cyclosporin capsules. The blood trough level can be measured by collecting blood immediately before administration of a specified calcineurin inhibitor (collecting blood and measuring at least 12 hours after the previous administration). In addition, the conversion value at the time of switching from tacrolimus to cyclosporine can be comprehensively determined by a doctor or the like in consideration of the number of days elapsed after surgery and the overall condition of the subject.

### 1) From day 0 to week 26 after liver transplantation calcineurin inhibitor dosage adjustment period)

In one embodiment, the administration can be initiated orally or by continuous intravenous injection from day 0 or day 1 after liver transplantation, and the blood trough level [target level: 8 to 12 ng/mL (tacrolimus) or 200 to 300 ng/mL (cyclosporine)] can be maintained until week 13 after liver transplantation. Until week 26 after liver transplantation, the blood trough level can be gradually reduced with the goal of 5 to 8 ng/mL for tacrolimus or 125 to 200 ng/mL for cyclosporine. When continuous intravenous injection is selected, the administration can be switched to oral administration (twice a day) from the time when the oral administration becomes possible.

In a case where tacrolimus is administered as a calcineurin inhibitor, a doctor or the like can, in principle, modify the dosage form to a sustained-release preparation one week before discharge, and administer the preparation (once a day). However, in a case where it is difficult to modify the dosage form to a sustained-release preparation due to medical reasons such as malabsorption of tacrolimus and medicament interaction, a normal preparation can be administered. When the normal preparation is administered, it is preferable to appropriately adjust the administration and dosage to maintain the target blood trough level, and to switch tacrolimus to the sustained-release preparation as soon as possible.

### 2) Week 26 or later after liver transplantation (dosage reduction period)

At week 26 after liver transplantation (dosage reduction 1), the blood trough level [target level: 35 ng/mL (tacrolimus or 75,125 ng/mL (cyclosporine)] can be maintained. In addition, in a case where tacrolimus cannot be modified to a sustained-release preparation during the dosage adjustment period of the calcineurin inhibitor, it is preferable to continue to adjust the administration and dosage appropriately to maintain the target blood trough level, and switch tacrolimus to a sustained-release preparation as soon as possible.

From week 39 after the liver transplantation (dosage reduction 2), the dosage at week 26 after the liver transplantation (dosage reduction 1) can be maintained, and the dosage can be decreased gradually such that the frequency of administration becomes, for example, 3 times per week at week 39 after the liver transplantation (dosage reduction 2), 2 times per week at week 52 after the liver transplantation (dosage reduction 3), 1 time per week at week 65 after the liver transplantation (dosage reduction 4), and withdrawal at week 78 after the liver transplantation (dosage reduction 5). When cyclosporine or tacrolimus normal preparation is administered, it is possible to appropriately adjust the administration and dosage using the blood trough level as an index to gradually reduce the dosage.

In one embodiment, the dosage can be modified when a doctor or the like determines that it is necessary to reduce the amount of the calcineurin inhibitor due to a side effect of the calcineurin inhibitor. Even in this case, the next visit schedule may not be modified.

In one embodiment, when the amount of the calcineurin inhibitor is reduced due to side effects, withdrawal from the immunosuppressant can be performed before reaching week 78 after liver transplantation (dosage reduction 5), according to the judgment of a doctor or the like.

### 3) Regulation for extending calcineurin inhibitor dosage reduction timing

In one embodiment, the timing of dosage reduction can be extended by a total of 13 weeks in a case where the doctor or the like determines that dosage reduction of the calcineurin inhibitor is impossible or that a modification in the medicament (tacrolimus to cyclosporine) is necessary, depending on the overall condition or the like of the subject. The number of times of dosage reduction extension is not limited, but in a case where the total extension period exceeds 13 weeks, dosage reduction for the purpose of withdrawing the immunosuppressant can be discontinued at that time, and the subject can be treated as necessary.

### <Whether or not patient continues to withdrawal at week 52 after withdrawal from immunosuppressants (calcineurin inhibitors) (evaluation of achievement of Operational tolerance)>

In one embodiment, the overall condition of the subject and blood biochemical test value can be observed over time for 52 weeks after the withdrawal from the immunosuppressant (calcineurin inhibitor), and the withdrawal from the immunosuppressant can be finally confirmed based on the liver biopsy and the like at week 52 after the withdrawal from the immunosuppressant. In addition, in one embodiment, in a case where a state where a stable liver function and no pathologically treatable rejection reaction are confirmed by liver biopsy continues for 52 weeks or more after the withdrawal from immunosuppressants, the case can be determined as having achieved operational tolerance.

### <Regular confirmation of rejection reaction, diagnosis and treatment, and subsequent regular confirmation of response rejection (liver biopsy)>

According to one aspect of the present disclosure, there is provided a method for confirming presence or absence of a rejection reaction by regular liver biopsies in immune tolerance therapy, the method including: a step of performing histological diagnosis on samples obtained by collecting liver tissue for control from a transplanted liver on the day of liver transplantation and samples obtained from regular liver biopsies after liver transplantation; and a step of determining that "there is a pathologically treatable rejection reaction", immediately initiating treatment for the rejection reaction, and discontinuing immune tolerance therapy, in a case where results of overall evaluation of acute rejection reaction satisfy any of the predetermined discontinuation criteria as a result of the histological diagnosis.

In addition, according to another aspect of the present disclosure, there is provided a method for confirming presence or absence of a rejection reaction by regular liver biopsies in immune tolerance therapy, the method including: a step of performing histological diagnosis according to scoring based on Banff criteria (2016) and Venturi (2012) with respect to samples obtained by collecting liver tissue for control from a transplanted liver on the day of liver transplantation and samples obtained from regular liver biopsies at week 26 after liver transplantation and 4 weeks and 52 weeks after dosage reduction 5; and a step of determining that "there is a pathologically treatable rejection reaction", immediately initiating treatment for the rejection reaction, and discontinuing immune tolerance therapy, in a case where results of overall evaluation of acute rejection reaction satisfy "moderate or higher", results of overall evaluation of chronic rejection reaction satisfy "present", and a score of liver fibrosis satisfy "2 or higher" as a result of the histological diagnosis.

At the time of dosage reduction/withdrawal of the immunosuppressant, there may be presence of liver fibrosis or chronic rejection even when the liver function test is normal. Therefore, the presence or absence of rejection reaction can be confirmed by regular liver biopsy. In addition, the liver biopsy can be performed under hospitalization (one night) in consideration of the safety of the subject after the liver biopsy.

In one embodiment, regular liver biopsies can be performed 26 weeks after liver transplantation, 4 weeks after dosage reduction 5, and 52 weeks after liver transplantation. In addition, liver tissue for control can be collected from the transplanted liver on the day of liver transplantation. For central pathological diagnosis of rejection reaction, a single sample of liver tissue can be collected using a 16 G needle for liver biopsy, and histological diagnosis of rejection reaction can be performed.

In one embodiment, the histological diagnosis of the tissue sample collected by the liver biopsy can be performed according to the scoring based on the Banff criteria (2016) and Venturi (2012), and the following evaluation can be performed.

### (1) Banff criteria (2016)

### Acute rejection reaction

Overall evaluation is performed based on the extent of inflammation in the portal regions and around the central veins. Furthermore, portal region inflammation, inflammatory injury of the bile duct, and venous endotheliitis are scored according to the criteria in Table 3, and the total point is expressed as Rejection Activity Index RAI.

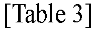

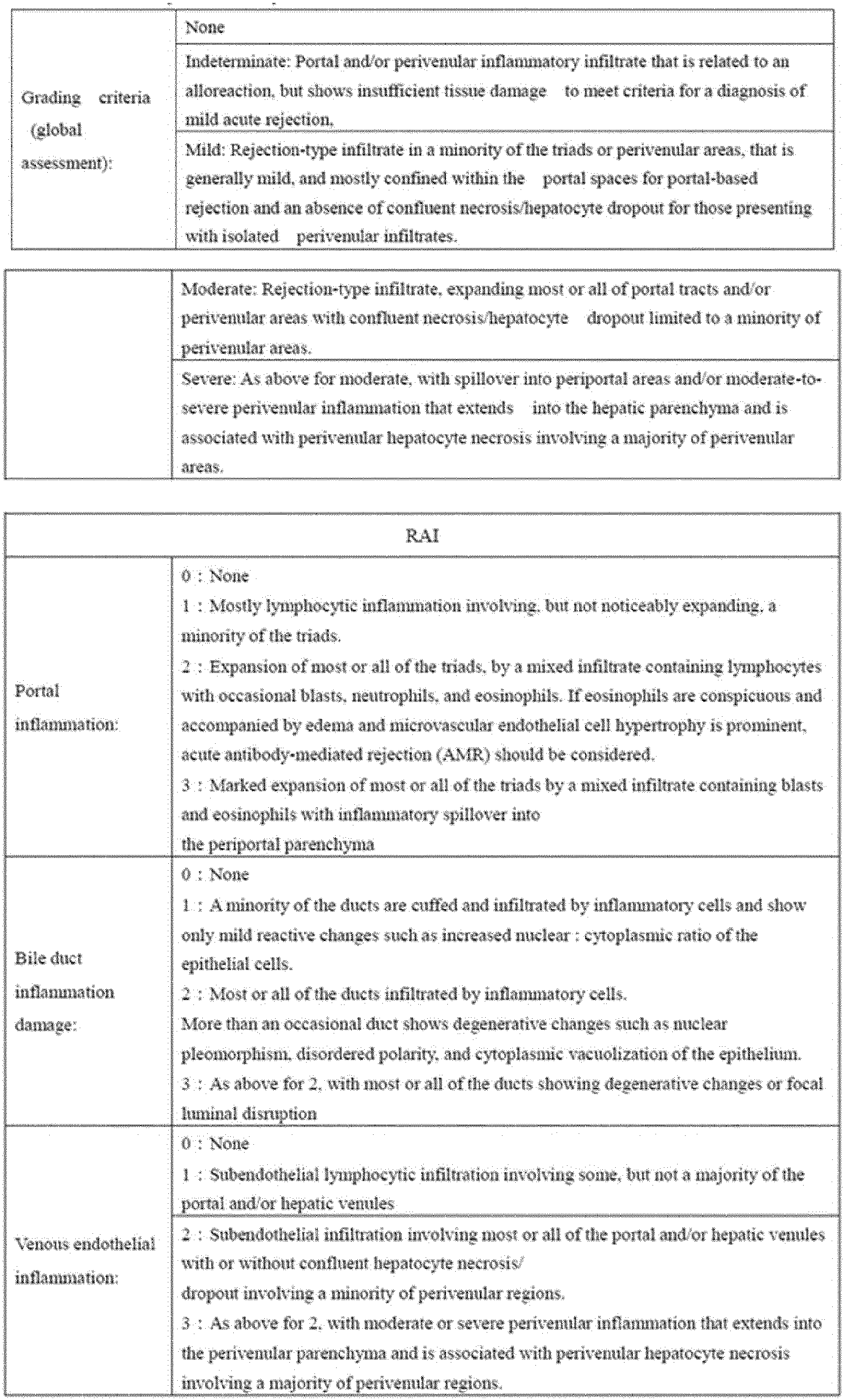

### Chronic rejection reaction

The degree of biliary loss, the degree of central venous fibrosis, the degree of arterial loss, and the results of other evaluations are evaluated according to the criteria in Table 4, and the overall evaluation is performed based on the evaluation results. The * mark indicates a structure that is usually observed only in the excised liver.

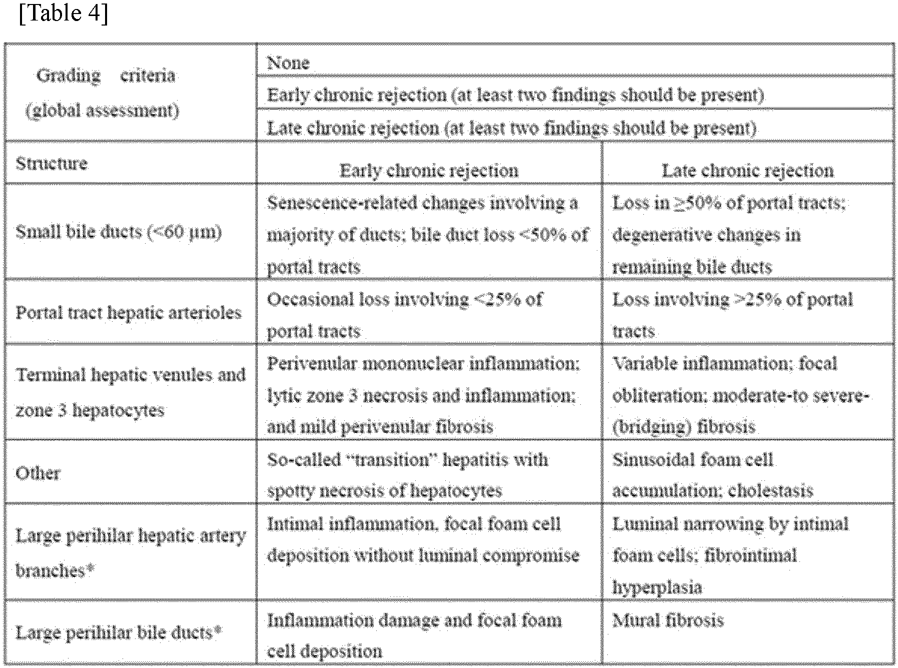

### (2) Scoring based on Venturi (2012)

The degree of fibrosis of the transplanted liver is scored according to the criteria in Table 5.

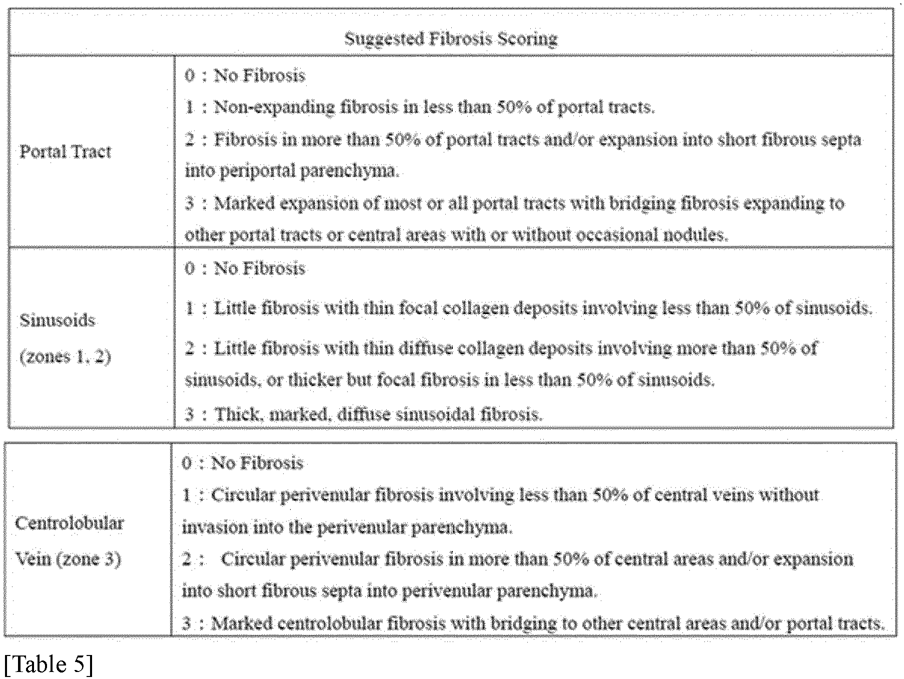

(3) When the result of histological diagnosis shows that the overall evaluation of acute rejection reaction is "moderate or more", the overall evaluation of chronic rejection is "present", and the score of hepatic fibrosis is "2 or more", it is determined that "there is a pathologically treatable rejection reaction", and treatment for rejection reaction can be immediately initiated.

### Confirmation of rejection reaction

Rejection reaction can be confirmed at the scheduled hospital visit and the non-scheduled hospital visit after liver transplantation. Hereinafter, a reference of the confirming procedure will be described.

### 1) Physical signs

Physical signs include fever, general fatigue, and the like.

### 2) Blood biochemical test

Any of T-Bil/AST/ALT/y-GTP increases by two or more times from the most recent visit data. However, when the liver function value increases by two or more times but is within the facility reference value, it is determined that the liver function value is stable.

### 3) Liver biopsy and pathological diagnosis

When abnormalities in the above 1) and 2) are observed and the rejection reaction is suspected, it is possible to perform a liver biopsy and histological diagnosis in a medical institution where the procedure is carried out.

### 4) Diagnosis of rejection reaction

The presence or absence of pathologically treatable rejection reaction is determined. The presence or absence of rejection reaction is determined from the result of 3).

When it is diagnosed that "there is a pathologically treatable rejection reaction", treatment for rejection reaction can be immediately initiated.

### References

The following references have been referred to as the basic technology in Examples and the like, and it is not an admission that these documents constitute related art with respect to the present disclosure. These contents are incorporated herein by reference.
1. Bashuda H, Kimikawa M, Seino K, Kato Y, Ono F, Shimizu A, et al. Renal allograft rejection is prevented by adoptive transfer of anergic T cells in nonhuman primates. The Journal of clinical investigation. 2005;115(7):1896-902.
2. Bashuda H, Shimizu A, Uchiyama M, Okumura K. Prolongation of renal allograft survival by anergic cells: advantages and limitations. Clin Transplant. 2010;24 Suppl 22:6-10.
3. Luo Z, Gotoh M, Grochowiecki T, Tanaka T, Kimura F, Kawashima H, et al. Anergic T cells generated in vitro suppress rejection response to islet allografts. Transplantation. 2000;69(10):2144-8.
4. Miyao T, Floess S, Setoguchi R, Luche H, Fehling HJ, Waldmann H, et al. Plasticity of Foxp3(+) T cells reflects promiscuous Foxp3 expression in conventional T cells but not reprogramming of regulatory T cells. Immunity. 2012;36(2):262-75.
5. Davies JK, Barbon CM, Voskertchian A, Nadler LM, Guinan EC. Ex vivo alloanergization with belatacept: a strategy to selectively modulate alloresponses after transplantation. Cell transplantation. 2012;21(9):2047-61.
6. Davies JK, Gribben JG, Brennan LL, Yuk D, Nadler LM, Guinan EC. Outcome of alloanergized haploidentical bone marrow transplantation after ex vivo costimulatory blockade: results of 2 phase 1 studies. Blood. 2008;112(6):2232-41.
7. Davies JK, Nadler LM, Guinan EC. Expansion of allospecific regulatory T cells after anergized, mismatched bone marrow transplantation. Science translational medicine. 2009;1(1):1-3.
8. Davies JK, Yuk D, Nadler LM, Guinan EC. Induction of alloanergy in human donor T cells without loss of pathogen or tumor immunity. Transplantation. 2008;86(6):854-64.

### (Note)

In the present specification, "or" is used when "at least one or more" of the items listed in the text can be employed. The same applies to "otherwise". In the present specification, in a case where "within a range of two values", the range includes the two values themselves.

References cited in the present specification, such as scientific literature, patents, patent applications, and the like, are hereby incorporated by reference in their entirety to the same extent as each was specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

Examples are described below. The handling of humans used in the following Examples was performed based on GCP, with the consent obtained, in accordance with the standards specified by the regulatory agencies and the Helsinki Declaration, in accordance with the Ethical Code specified by Juntendo University, in accordance with the ICH standards, as necessary. Compliance will be maintained with the standards proposed by the Helsinki Declaration and ICH, as well as with the various standards set forth by the Juntendo University Ethics Committee. As the reagents, specifically, the products described in Examples were used, but equivalent products of other manufacturers (Fujirebio, Sigma-Aldrich, and the like) can be substituted.

### (Example 1: MLR and IFN-y quantitative ELISA)

Frozen and stored donor-derived cells were thawed and adjusted to 2.0 x 10⁶ cells/ml with a culture medium. Similarly, frozen patient-derived cells were thawed and adjusted to 2.0 × 10⁶ cells/ml with a culture medium.

The cell products were adjusted to 2.0 cells/ml with a culture medium, and the cells were mixed at the following ratio.
1. Only Patient-derived Cells
2. Patient-derived cells : Donor-derived cells = 1 : 1
3. Patient-derived cells : Donor-derived cells : cell products = 1 : 1 : 1/2
4. Patient-derived cells : Donor-derived cells : cell products = 1 : 1 : 1/4
5. Patient-derived cells : Donor-derived cells : cell products = 1 : 1 : 1/8
6. Patient-derived cells : Donor-derived cells : cell products = 1 : 1 : 1/16

The cells were cultured at 37°C for 5 days, and the culture supernatant was collected. The culture supernatant was diluted 5-fold, and the amount of IFNg was measured by ELISA. Based on the IFNg concentration, the ratio of the group in the above 2. as 100% was calculated. The results are shown in FIGS. 1A to 1C.

FIGS. 1A, 1B, and 1C respectively show the results of three times of independent productions. The graph on the left side of each drawing shows the IFNg concentration measured by ELISA, and the graph on the right side shows the proportion in a case where the IFNg concentration in the culture supernatant of a group (Allo) in which only patient-derived cells and donor-derived cells were mixed and cultured is 100%.

In the production example of FIG. 1A, it is suggested that the release of IFNg cannot be suppressed even when the cell product is added, and the immunosuppressive ability of the cell product is low. In FIGS. 1B and 1C, the IFNg concentration decreases as the proportion of the cell product increases, and it is considered that the immunosuppressive ability of the cell product is high.

### (Example 2: living-donor liver transplantation and immunosuppression)

FIG. 2 shows an overall image for achieving dosage reduction of an immunosuppressant for a patient after living-donor liver transplantation, using an induced regulatory T cell preparation according to the present disclosure. Lymphocytes are collected from a donor and a patient (recipient), respectively, and co-cultured with CD80 and CD86 antibodies to obtain an induced regulatory T cell preparation of the present disclosure. As the CD80 and CD86 antibodies, antibodies disclosed in WO2019/245037 are used. Thereafter, the induced regulatory T cell preparation of the present disclosure is administered to a patient after living-donor liver transplantation to reduce the dosage of the immunosuppressant.

The schedule from living-donor liver transplantation to dosage reduction of the immunosuppressant is performed as shown in FIGS. 3A to 3C. The patient schedule is shown in FIGS. 3A and 3B, and the donor schedule is shown in FIG. 3C.

The administration and dosage reduction of the immunosuppressant (dosage adjustment and dosage reduction period of the immunosuppressant) are performed according to the schedule of FIG. 4. Thereafter, it is confirmed whether or not withdrawal of the immunosuppressant has been achieved.

### (Example 3: immune tolerance prediction)

About 40 mL of blood is collected from a patient and a donor before transplantation, and blood cells are separated. Some blood cells are frozen and used for the subsequent MLR test.

The induced regulatory T cells of the present disclosure are obtained by co-culturing patient-derived blood cells and radiation-irradiated donor-derived blood cells with CD80 and CD86 antibodies. As the CD80 and CD86 antibodies, antibodies disclosed in WO2019/245037 are used.

The suppressive ability of the obtained induced regulatory T cells is evaluated by MLR.

By performing the above test, it becomes possible to predict the reactivity and/or suppressive ability of the produced induced regulatory T cells to the donor cell before actually producing the induced regulatory T cells. Accordingly, it is possible to use, as an index, whether or not to produce induced regulatory T cells or whether or not to administer induced regulatory T cells.

### (Example 4: living-donor liver transplantation and immunosuppression)

Immunosuppression of the patient after living-donor liver transplantation was confirmed.

The patient was administered with the induced regulatory T cells on day 10 after living-donor liver transplantation and did not develop a serious adverse event associated with the administration. According to the protocol, the immunosuppressant could be reduced from the steroid, calcineurin inhibitor (tacrolimus), and antimetabolite (CellCept) to one type of tacrolimus sustained-release preparation within 1 month after transplantation, and the patient was discharged from the hospital on day 36 after transplantation.

For 6 months after transplantation, no graft injury was observed clinically or pathologically, and thus the stage proceeded to a stage of actively reducing the dosage of the immunosuppressant. It was possible to reduce the dosage of the immunosuppressant in the order of 7 times/week → 3 times/week → 2 times/week → 1 time/week → 0 times/week every 3 months and completely interrupt the dosage reduction on day 628 after transplantation. In addition, as a result of performing a liver biopsy at week 4 after discontinuing the immunosuppressant and performing evaluation according to the Banff criteria, no findings of potential pathological rejection reaction were observed, and it was suggested that immune tolerance could be safely induced.

### (Note)

While the present disclosure has been illustrated using preferred embodiments of the present disclosure, it is understood that the scope of the present disclosure should be interpreted only by the claims. It is understood that the patents, patent applications and other documents cited in the present specification are to be incorporated by reference in their content to this specification, as the content thereof is specifically described in the present specification. The present application claims priority to Japanese Patent Application No. 2022-60165 filed on March 31, 2022 to the Japanese Patent Office, the entire contents of which are incorporated herein by reference in the same manner as if the entire contents of the present application were composed.

### Industrial Applicability

The present disclosure provides a method for evaluating the quality of an induced regulatory T cell preparation, and provides various technologies related to immune tolerance therapy.

## Claims

1. A method for evaluating quality of a medicament for achieving immune tolerance, which is administered to a patient who has undergone living-donor liver transplantation, the method comprising:
(a) a step of mixing and culturing peripheral blood lymphocytes collected from a patient before the living-donor liver transplantation and peripheral blood lymphocytes collected from a donor of the living-donor liver transplantation;
(b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient; and
(c) a step of measuring cytokine production in steps (a) and (b), wherein
in a case where the cytokine production in step (b) has changed compared to step (a), the medicament is considered to be a medicament for immune tolerance therapy.

2. The method according to claim 1, wherein the patient has undergone administration of an immunosuppressant.

3. The method according to claim 1 or 2, wherein the medicament is an inhibitory factor that inhibits an interaction of CD80 and/or CD86 expressed on a cell surface of a certain cell with CD28 expressed on a cell surface of another cell.

4. The method according to any one of claims 1 to 3, wherein the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

5. The method according to any one of claims 1 to 4, wherein the immune tolerance therapy for the patient is modified in a case where the cytokine production in step (b) changes compared to step (a).

6. The method according to any one of claims 1 to 5, wherein
(i) in a case where pro-inflammatory cytokine production in step (b) increases compared to step (a), an amount of immunosuppressant administered to the patient is increased and/or the immune tolerance therapy for the patient is discontinued; and/or
(ii) in a case where anti-inflammatory cytokine production in step (b) decreases compared to step (a), an amount of immunosuppressant administered to the patient is increased and/or the immune tolerance therapy for the patient is discontinued.

7. The method of any one of claims 1 to 6, wherein the peripheral blood lymphocytes are stained with CFSE.

8. The method according to any one of claims 1 to 7, wherein in steps (a) and (b), B cells collected from the donor are used instead of peripheral blood lymphocytes collected from the donor.

9. The method according to any one of claims 1 to 8, wherein the method is carried out within a period from at least about day 3 to day 6 after the living-donor liver transplantation.

10. The method according to any one of claims 1 to 9, wherein the cytokine includes IFNγ, TNF, IL-2, IL-12, IL-15, IL-17, IL-18, IL-10, and/or TGFβ.

11. A method for achieving immune tolerance in a patient, the method comprising:
a step of collecting lymphocytes from a donor by apheresis;
a step of collecting lymphocytes from the patient by apheresis;
a step of subjecting a liver or a part of the liver derived from the donor to living-donor liver transplantation for the patient;
a step of performing, on the patient after the living-donor liver transplantation,
continuous administration of corticosteroids, antimetabolites, and a first immunosuppressant, and
immune monitoring for rejection reaction to the living-donor liver transplantation and/or regular liver biopsies;
a step of administering a second immunosuppressant to the patient after the living-donor liver transplantation;
a step of administering a medicament for achieving immune tolerance in the patient to the patient after the administration of the second immunosuppressant; and
a step of gradually reducing dosages of the corticosteroids, the antimetabolites, and the first immunosuppressant.

12. The method according to claim 11, wherein
based on the immune monitoring for the rejection reaction and/or the regular liver biopsies, it is determined whether achievement of immune tolerance is confirmed or whether transition to conventional immunosuppressive therapy is performed,
an overall condition of the patient and/or blood biochemical test values are observed over time for a predetermined period after discontinuation of the administration of the first immunosuppressant, and the discontinuation of the administration of the first immunosuppressant is finally determined by performing a liver biopsy at a time point when a predetermined period has elapsed after the discontinuation of the administration of the first immunosuppressant, and
further, in a case where stable liver function values and a state where no pathologically treatable rejection reaction is confirmed in the liver biopsy continue for a predetermined period or more after the discontinuation of the administration of the first immunosuppressant, it is determined that the patient has achieved operational tolerance.

13. A method for achieving immune tolerance in a patient, the method comprising:
a step of collecting lymphocytes from a donor by apheresis 3 to 14 days before transplantation;
a step of collecting lymphocytes from the patient by apheresis 1 day before transplantation;
a step of subjecting a liver or a part of the liver derived from the donor to living-donor liver transplantation for the patient;
a step of performing, on the patient after the living-donor liver transplantation,
continuous administration of corticosteroids, antimetabolites, and calcineurin inhibitors, and
immune monitoring for rejection reaction to the living-donor liver transplantation and/or regular liver biopsies;
a step of administering 20 to 50 mg/kg of cyclophosphamide to the patient on day 4 to day 6 after the liver transplantation;
a step of administering a medicament for achieving immune tolerance in the patient to the patient at any time point between day 9 and day 11 after the liver transplantation; and
a step of reducing a dosage of the calcineurin inhibitor at least 13 weeks after the transplantation, wherein
based on the immune monitoring for the rejection reaction and/or the regular liver biopsies, it is determined whether achievement of immune tolerance is confirmed or whether transition to conventional immunosuppressive therapy is performed,
an overall condition of the patient and/or blood biochemical test values are observed over time for at least 52 weeks after discontinuation of the administration of the calcineurin inhibitor, and the discontinuation of the administration of the calcineurin inhibitor is finally determined by performing a liver biopsy at the time point of at least week 52 after the discontinuation of the administration of the calcineurin inhibitor, and
further, in a case where stable liver function values and a state where no pathologically treatable rejection reaction is confirmed by the liver biopsy continue for more than 52 weeks after the discontinuation of the administration of the calcineurin inhibitor, it is determined that the patient has achieved operational tolerance.

14. The method according to any one of claims 11 to 13, wherein the administration of the corticosteroids and antimetabolites to the patient is discontinued at least within 4 weeks after the living-donor liver transplantation.

15. The method according to any one of claims 11 to 14, wherein the administration of the corticosteroids and antimetabolites to the patient is discontinued at least within 26 weeks after the living-donor liver transplantation.

16. The method according to any one of claims 11 to 15, wherein the administration of the calcineurin inhibitors to the patient is discontinued at least within 78 weeks after the living-donor liver transplantation.

17. The method according to any one of claims 11 to 16, wherein the medicament is an inhibitory factor that inhibits an interaction of CD80 and/or CD86 expressed on a cell surface of a certain cell with CD28 expressed on a cell surface of another cell.

18. The method according to any one of claims 11 to 17, wherein the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

19. A method for reducing dosage of an immunosuppressant in immune tolerance therapy, the method comprising:
(1) a step of administering corticosteroids according to the following schedule of administration at the time of reperfusion, and
administration after liver transplantation and dosage reduction, termination of the administration within a predetermined period, and termination of the administration using an alternative dosage reduction method in a case where the administration is not terminated within the predetermined period;
(2) a step of administering antimetabolites according to the following schedule of administration after liver transplantation,
termination of the administration within a predetermined period after liver transplantation,
termination of the administration using an alternative dosage reduction method in a case where the administration is not terminated within a predetermined period after liver transplantation, and
if necessary, allowing an increase in dosage with the goal of one week after liver transplantation, and in this case, gradual dosage reduction; and
(3) a step of administering a first immunosuppressant according to the following schedule of
(3-1) a predetermined period after liver transplantation (dosage adjustment period), in which
administration is initiated on the day of liver transplantation or day 1 after liver transplantation, targeting each of the following blood trough levels such as
a predetermined blood trough level for a first predetermined period from the initiation of the administration to after liver transplantation, and
a predetermined blood trough level for a second predetermined period following the first predetermined period after liver transplantation,
(3-2) week 26 or later after liver transplantation (dosage reduction period of the first immunosuppressant), in which
in a first predetermined period after liver transplantation (dosage reduction 1), the administration and dosage are adjusted to maintain a predetermined blood trough level, and
after the second predetermined period after liver transplantation (dosage reduction 2), the dosage of (dosage reduction 1) is maintained, and a frequency of administration is gradually reduced according to a dosage reduction schedule, and
(3-3) if necessary, the dosage reduction period of the first immunosuppressant is extended, wherein
each dosage reduction is carried out in a case where it is confirmed that no rejection reaction is present.

20. A method for reducing dosage of an immunosuppressant in immune tolerance therapy, the method comprising:
(1) a step of administering corticosteroids according to the following schedule of administration of 1,000 mg at the time of reperfusion,
administration of 20 mg/day from day 1 for one week after liver transplantation,
dosage reduction by 5 mg/day every week and termination of the administration within 4 weeks after liver transplantation, and
termination of the administration by week 26 after liver transplantation using an alternative dosage reduction method in a case where the administration is not terminated within 4 weeks after liver transplantation;
(2) a step of administering antimetabolites according to the following schedule of
administration of 500 mg/day in total divided into two doses per day from day 1 after liver transplantation,
termination of the administration within 4 weeks after liver transplantation,
termination of the administration by week 26 after liver transplantation using an alternative dosage reduction method in a case where the administration is not terminated within 4 weeks after liver transplantation, and
if necessary, allowing an increase in dosage to 1,000 to 2,000 mg/day with the goal of one week after liver transplantation, and in this case, gradual dosage reduction by 500 mg/day; and
(3) a step of administering calcineurin inhibitors according to the following schedule of
(3-1) a period from day 0 after liver transplantation to week 26 after liver transplantation (calcineurin inhibitor dosage adjustment period), in which
administration is initiated orally or by continuous intravenous infusion on the day of liver transplantation or day 1 after liver transplantation, targeting each of the following blood trough levels such as
8 to 12 ng/mL of tacrolimus or 200 to 300 ng/mL of cyclosporine during a period from initiation of administration to week 13 after liver transplantation, and
5 to 8 ng/mL of tacrolimus or 125 to 200 ng/mL of cyclosporine during a period from week 13 after liver transplantation to week 26 after liver transplantation,
(3-2) week 26 or later after liver transplantation (calcineurin inhibitor dosage reduction period), in which
in week 26 after liver transplantation (dosage reduction 1), the administration and dosage are adjusted to maintain a blood trough level of 3 to 5 ng/mL of tacrolimus or 75 to 125 ng/mL of cyclosporine with a frequency of administration to once a day, and
from week 39 after liver transplantation (dosage reduction 2), the dosage from week 26 after liver transplantation (dosage reduction 1) is maintained, and a frequency of administration is gradually reduced according to a calcineurin inhibitor dosage reduction schedule (dosage reduction 2: three times a week, dosage reduction 3: twice a week, dosage reduction 4: once a week, dosage reduction 5: withdrawal), and
(3-3) if necessary, the calcineurin inhibitor dosage reduction period is extended for a total of 13 weeks, wherein
each dosage reduction is carried out in a case where no rejection reaction is confirmed.

21. The method according to claim 19 or 20, wherein the presence of the rejection reaction is determined by
(1) physical signs such as fever or general fatigue, and/or
(2) an increase by two or more times from the most recent visit data in any of blood biochemical tests such as T-Bil, AST, ALT, or γ-GTP.

22. A method for confirming presence or absence of a rejection reaction by regular liver biopsies in immune tolerance therapy, the method comprising:
a step of performing histological diagnosis on samples obtained by collecting liver tissue for control from a transplanted liver on the day of liver transplantation and samples obtained from regular liver biopsies after liver transplantation; and
a step of determining that "there is a pathologically treatable rejection reaction", immediately initiating treatment for the rejection reaction, and discontinuing immune tolerance therapy, in a case where results of overall evaluation of acute rejection reaction satisfy any of the predetermined discontinuation criteria as a result of the histological diagnosis.

23. A method for confirming presence or absence of a rejection reaction by regular liver biopsies in immune tolerance therapy, the method comprising:
a step of performing histological diagnosis according to scoring based on Banff criteria (2016) and Venturi (2012) with respect to samples obtained by collecting liver tissue for control from a transplanted liver on the day of liver transplantation and samples obtained from regular liver biopsies at week 26 after liver transplantation and 4 weeks and 52 weeks after dosage reduction 5; and
a step of determining that "there is a pathologically treatable rejection reaction", immediately initiating treatment for the rejection reaction, and discontinuing immune tolerance therapy, in a case where results of overall evaluation of acute rejection reaction satisfy "moderate or higher", results of overall evaluation of chronic rejection reaction satisfy "present", and a score of liver fibrosis satisfy "2 or higher" as a result of the histological diagnosis.

24. A method for achieving immune tolerance in a patient who has undergone living-donor liver transplantation using a medicament for achieving immune tolerance evaluated in quality, the method comprising:
(a) a step of mixing and culturing peripheral blood lymphocytes collected from the patient before the living-donor liver transplantation and peripheral blood lymphocytes collected from a donor of the living-donor liver transplantation;
(b) a step of mixing and culturing peripheral blood lymphocytes collected from the patient before the living-donor liver transplantation, peripheral blood lymphocytes collected from the donor of the living-donor liver transplantation, and the medicament for achieving immune tolerance in the patient;
(c) a step of measuring cytokine production in steps (a) and (b);
(d) a step of performing, on the patient after the living-donor liver transplantation,
continuous administration of corticosteroids, antimetabolites, and a first immunosuppressant, and
immune monitoring for rejection reaction to the living-donor liver transplantation and/or regular liver biopsies;
(e) a step of administering a single dose of a second immunosuppressant to the patient after the living donor liver transplant; and
(f) a step of administering a medicament that has changed cytokine production in step (b) compared to step (a), to the patient after the administration of the second immunosuppressant; and
a step of gradually reducing dosages of the corticosteroids, the antimetabolites, and the first immunosuppressant.

25. The method according to claim 24, wherein the patient has undergone administration of an immunosuppressant.

26. The method according to claim 24 or 25, wherein the medicament is an inhibitory factor that inhibits an interaction of CD80 and/or CD86 expressed on a cell surface of a certain cell with CD28 expressed on a cell surface of another cell.

27. The method according to any one of claims 24 to 26, wherein the medicament is an induced regulatory T cell preparation, and the induced regulatory T cell preparation is obtained by co-culturing the peripheral blood lymphocytes derived from the patient and the donor of the living-donor liver transplantation in the presence of CD80 antibodies and/or CD86 antibodies.

28. The method according to any one of claims 24 to 27, wherein the immune tolerance therapy for the patient is modified in a case where the cytokine production in step (b) changes compared to step (a).

29. The method according to any one of claims 24 to 28, wherein
(i) in a case where pro-inflammatory cytokine production in step (b) increases compared to step (a), an amount of immunosuppressant administered to the patient is increased and/or the immune tolerance therapy for the patient is discontinued; and/or
(ii) in a case where anti-inflammatory cytokine production in step (b) decreases compared to step (a), an amount of immunosuppressant administered to the patient is increased and/or the immune tolerance therapy for the patient is discontinued.

30. The method of any one of claims 24 to 29, wherein the peripheral blood lymphocytes are stained with CFSE.

31. The method according to any one of claims 24 to 30, wherein in steps (a) and (b), B cells collected from the donor are used instead of peripheral blood lymphocytes collected from the donor.

32. The method according to any one of claims 24 to 31, wherein the method is carried out within a period from at least about day 3 to day 6 after the living-donor liver transplantation.

33. The method according to any one of claims 24 to 32, wherein the cytokine includes IFNγ, TNF, IL-2, IL-12, IL-15, IL-17, IL-18, IL-10, and/or TGFβ.
